# EUROPEAN PATENT SPECIFICATION

(11) **EP 1 943 275 B1**
(45) Date of publication and mention of the grant of the patent: **16.06.2010**
(21) Application number: 06794924.8
(22) Date of filing: 27.10.2006
(51) Int. Cl.: C07K 14/605, A61K 38/26

(54) **INSULINOTROPIC COMPOUNDS AND USES THEREOF**
INSULINOTROPE VERBINDUNGEN UND ANWENDUNGEN DAVON
COMPOSÉS INSULINOTROPES ET APPLICATIONS

(30) Priority: 01.11.2005 GB 0522295; 11.08.2006 GB 0616061
(43) Date of publication of application: 16.07.2008
(73) Proprietor: Activotec SPP Limited, Shakespeare House 42 Newmarket Road Cambridge, Cambridgeshire CB5 8EP (GB)
(72) Inventor: KANDA, Patrick, Southampton SO16 OTD (GB)
(74) Representative: Duncan, Garreth Andrew
(86) International application number: PCT/GB2006/004018
(87) International publication number: WO 2007/051987

(56) References cited:
- WO-A-00/34332
- WO-A-00/69911
- WO-A-91/11457
- WO-A-2004/005342
- WO-A2-03/033671
- US-A- 5 981 488
- US-B1- 6 620 910
- US-B1- 6 849 708
- US-B2- 6 703 365
- XIAO Q ET AL: "Biological activities of glucagon-like peptide-1 analogues in vitro and in vivo" BIOCHEMISTRY, AMERICAN CHEMICAL SOCIETY. EASTON, PA, US, vol. 40, no. 9, 6 March 2001 (2001-03-06), pages 2860-2869, XP002277645 ISSN: 0006-2960

## Description

### Field of the Invention

The present invention relates to certain peptide-like compounds, to processes for their preparation, compositions comprising them and methods of treatment of the human or animal body employing them.

### Background

Type II diabetes (non-insulin dependent diabetes mellitus - NIDDM) is a condition characterized by a resistance to insulin action in peripheral tissues such as muscle, adipose and liver, and by a progressive failure of the ability of the islet β-cells to secrete insulin. Because current therapies do not halt the progression of β-cell failure, virtually all NIDDM patients eventually require insulin to control blood glucose levels. The most commonly prescribed therapeutics for such patients are the sulfonylureas, a class of drugs that stimulate insulin secretion. Each year, 10-20% of the patients receiving sulfonylurea therapy fail to maintain acceptable blood glucose levels, and switch to insulin therapy.

Insulin therapy, however, is undesirable from a variety of points of view. Firstly, it has a narrow therapeutic index. This leads to poor control of blood glucose levels, since most patients and physicians err in favour of high glucose levels rather than risk hypoglycaemia and coma.

Secondly, assessment of the appropriate dosage is difficult. The factors to be taken into account include the amount of food consumed, the interval between meals, the amount of physical exercise, and the prevailing blood glucose level (the determination of which requires blood glucose monitoring).

Thirdly, administration of insulin is inconvenient because it must be given parenterally.

For these reasons, satisfactory control of blood glucose levels is frequently not achieved in patients receiving insulin therapy.

The hormone glucagon-like peptide-1 (7-37) (GLP-1) is released by intestinal L cells in response to ingested nutrients and acts to promote glucose-dependent insulin secretion ensuring efficient postprandial glucose homeostasis. This hormone binds the GLP-1 receptor present on a number target tissues including the lungs, pancreatic β cells, brain, muscle, and gut, achieving normal glucose levels in five separate but complementary ways. The peptide is synthesised as a prohormone in which the N terminal 6 amino acids are processed to yield the biologically active GLP-1 7-37 sequence:

Unfortunately, the beneficial actions of GLP-1 which give this hormone many of the desirable properties of an antidiabetic drug are short lived due to N terminal degradation by dipeptidylpeptidase IV (DPP IV) and rapid clearance by renal filtration.

DPP IV cleavage occurs between Ala₈ and Glu₉, completely inactivating it and resulting in a circulating half-life of less than 2 minutes for the active form of GLP-1. Various strategies have been employed to protect GLP-1 against both renal clearance and DPP-IV cleavage in efforts to produce a more stable, enduring antidiabetic drug. They include amino acid substitutions at Ala₈ and elsewhere, covalent attachment of fatty acids, and the linking of GLP-1 to albumin. Modification of GLP-1 through fatty acid attachment confers protection from renal clearance and DPP-IV activity from its resulting association in the bloodstream with albumin. A therapeutic candidate for treating Type II diabetes based on this modification has emerged and is currently undergoing clinical trials. In addition, a number of small molecule DPP IV inhibitors are being developed for oral delivery to prolong the activity of endogenously secreted GLP-1.

### Prior Art

US5981488 discloses GLP-1 analogues of the formula: or a pharmacuetically accetable salt thereof, wherein: R₁ is selected from the group consisting of His, D-histidine, desamino-histidine, 2-amino-histidine, β-hydroxy-histidine, homohistidine, α-fluoromethyl-histidine, and α-methyl-histidine; X is selected from the group consisting of Met, Asp, Lys, Thr, Leu, Asn, Gln, Phe, Val, and Tyr; Y and Z are independently selected from the group consisting of Glu, Gln, Ala, Thr, Ser, and Gly, and R₂ is selected from the group consisting of NH₂, and Gly-OH; provided that, if R₁ is His, X is Val, Y is Glu, and Z is Glu, then R₂ is NH₂.

The GLP-1 analogues are stated to have an increased duration of action and resistance to DPP-IV.

US6620910 discloses GLP-1 analogues of the formula wherein Z₁ is substituent of the terminal amino group of the peptide; Z₂ is a substituent of the terminal carbonyl group of the peptide; and X₁ to X₁₄ each represents, independently of the others a natural or non-natural amino acid residue, having the D or L configuration.

The compounds are stated to have agonist character in relation to ^{t}GLP-1 receptors, in addition to increased metabolic stability and duration of action when compared to the natural peptide.

US6703365 discloses peptides of the formula wherein: R₁ is selected from the group consisting of L-histidine, D-histidine, desamino-histidine, 2-amino-histidine, β-hydroxy-histidine, homohistidine, α-fluoromethyl-histidine, and α-methyl-histidine; X is selected from the group consisting of Ala, Gly, Val, Thr, Ile, and alpha-methyl-Ala; Y is selected from the group consisting of Glu, Gin, Ala, Thr, Ser, and Gly; Z is selected from the group consisting of Glu, Gln, Ala, Thr, Ser, and Gly; R₂ is selected from the group consisting of NH₂, and Gly-OH; providing that the compound has an isoelectric point in the range from about 6.0 to about 9.0 and further providing that when R₁ is His, X is Ala, Y is Glu, and Z is Glu, R₂ must be NH₂.

The peptides are said to have increased stability to storage and *in vivo.*

US6849708 discloses insulinotropic peptides comprising a fragment of GLP-1 and derivatives thereof.

A problem that remains is the provision of therapeutic agents for the treatment of diabetes.

A further problem that remains is the provision of compounds having GLP-1 like activity while having superior stability compared with naturally occurring GLP-1.

A further problem that remains is the provision of GLP-1 analogues having increased resistance to degradation by DPP-IV.

The present invention addresses problems of the prior art.

### Summary of the Invention

According to a first aspect, there is provided an insulinotropic compound of formula (XXV), (XXVII) or (XXVIII): 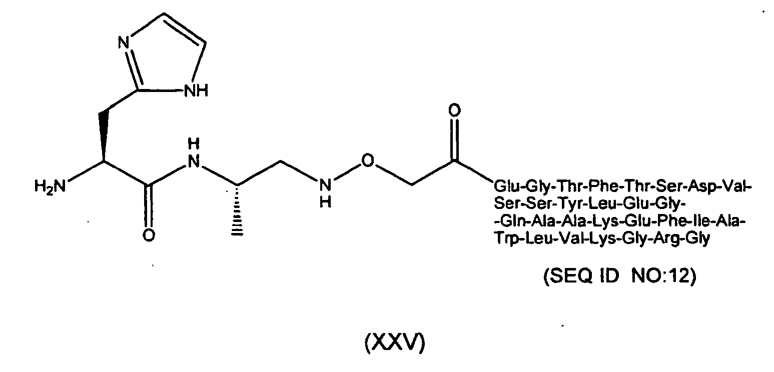 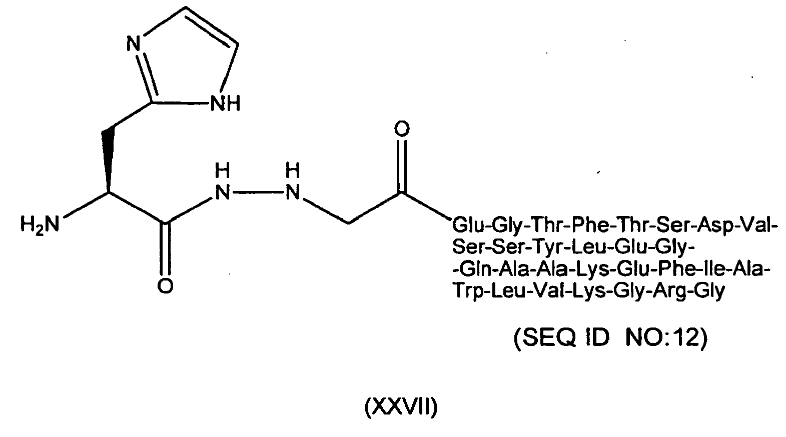 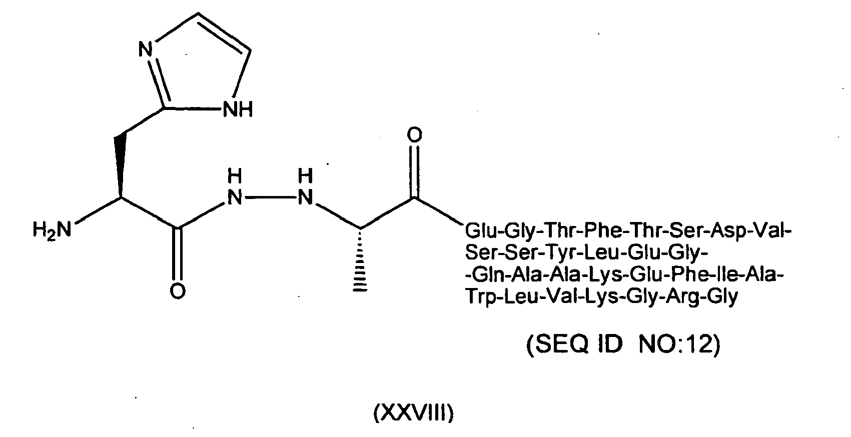 or a pharmeuceutically acceptable salt form thereof.

According to a second aspect, there is provided a compound of the present invention for use as a pharmaceutical.

According to a third aspect, there is provided a pharmaceutical composition comprising a compound of the present invention together with a pharmaceutically acceptable carrier or excipient.

According to a fourth aspect, there is provided a compound of the present invention for use in the treatment of a human or animal suffering from a condition selected from hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers, comprising administering to said mammal a compound of formula (I) or a composition comprising such a compound.

According to a fifth aspect, there is provided the use of a compound of the present invention or a composition comprising such a compound in the preparation of a medicament for the treatment of a condition selected from hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers.

### Detailed Description of the Invention

The compounds of the invention have insulinotropic activity. As used herein, the term "insulinotropic activity" refers to the property of a compound to stimulate the synthesis or expression of the hormone insulin.

Preferably, the compounds of the invention have insulinotropic activity that is at least 10 % of that of GLP-1 (7-36). More preferably, the compounds of the invention have insulinotropic activity that is at least 20 % of that of GLP-1 (7-36). More preferably, the compounds of the invention have insulinotropic activity that is at least 50 % of that of GLP-1 (7-36). More preferably, the compounds of the invention have insulinotropic activity that is at least 80 % of that of GLP-1 (7-36). More preferably, the compounds of the invention have insulinotropic activity that is at least equal to that of GLP-1 (7-36).

Insulinotropic activity in this context may be measured using a suitable assay. The insulinotropic property of a compound may be determined by providing that compound to animal cells, or injecting that compound into animals and monitoring the release of immunoreactive insulin (IRI) into the media or circulatory system of the animal, respectively. The presence of IRI is detected through the use of a radioimmunoassay which can specifically detect insulin. Although any radioimmunoassay capable of detecting the presence of IRI may be employed, it is preferable to use a modification of the assay method of Albano, J. D. M., et al., (Acta Endocrinol. 70, 487-509 (1972)). In this modification, a phosphate/albumin buffer with a pH of 7.4 was employed. The incubation was prepared with the consecutive addition of 500 µl of phosphate buffer, 50 µl of perfusate sample or rat insulin standard in perfusate, 100 µl of anti-insulin antiserum (Wellcome Laboratories; 1:40,000 dilution), and 100 µl of [¹²⁵l] insulin, giving a total volume of 750 µl in a 10*75-mm disposable glass tube. After incubation for 2-3 days at 4° C., free insulin was separated from antibody-bound insulin by charcoal separation. The assay sensitivity was 1-2 µU/ml. In order to measure the release of IRI into the cell culture medium of cells grown in tissue culture, one preferably incorporates radioactive label into proinsulin. Although any radioactive label capable of labelling a polypeptide can be used, it is preferable to use ³H leucine in order to obtain labelling of proinsulin. Labeling can be done for any period of time sufficient to permit the formation of a detectably labelled pool of proinsulin molecules; however, it is preferable to incubate cells in the presence of radioactive label for a 60-minute time period. Although any cell line capable of expressing insulin can be used for determining whether a compound has an insulinotropic effect, it is preferable to use rat insulinoma cells, and especially RIN-38 rat insulinoma cells. Such cells can be grown in any suitable medium; however, it is preferable to use DME medium containing 0.1 % BSA and 25 mM glucose.

The insulinotropic property of a compound may also be determined by pancreatic infusion. The *in situ* isolated perfused rat pancreas preparation was a modification of the method of Penhos, J. C., et al. (Diabetes 18, 733-738 (1969)). In accordance with such a method, fasted rats (preferably male Charles River strain albino rats), weighing 350-600 g, are anesthetized with an intraperitoneal injection of Amytal Sodium (Eli Lilly and Co., 160 ng/kg). Renal, adrenal, gastric, and lower colonic blood vessels are ligated. The entire intestine is resected except for about four cm of duodenum and the descending colon and rectum. Therefore, only a small part of the intestine is perfused, thus minimizing possible interference by enteric substances with glucagon-like immunoreactivity. The perfusate is preferably a modified Krebs-Ringer bicarbonate buffer with 4% dextran T70 and 0.2% bovine serum albumin (fraction V), and is preferably bubbled with 95% O₂ and 5% CO₂. A nonpulsatile flow, four-channel roller-bearing pump (Buchler polystatic, Buchler Instruments Division, Nuclear-Chicago Corp.) is preferably used, and a switch from one perfusate source to another is preferably accomplished by switching a three-way stopcock. The manner in which perfusion is performed, modified, and analyzed preferably follows the methods of Weir, G. C., et al., (J. Clin. Investigat. 54:1403-1412 (1974)), which is hereby incorporated by reference.

### DPP IV resistance

Preferably, the compounds of the invention show increased resistance to deactivation by DPP IV compared with, natural GLP-1. Resistance to DPP IV deactivation is suitably measured for example by the technique described in in Green, B.D. et al, Biological Chemistry, 385,169-177,(2004).

### Compounds

The compounds of the invention are the following: 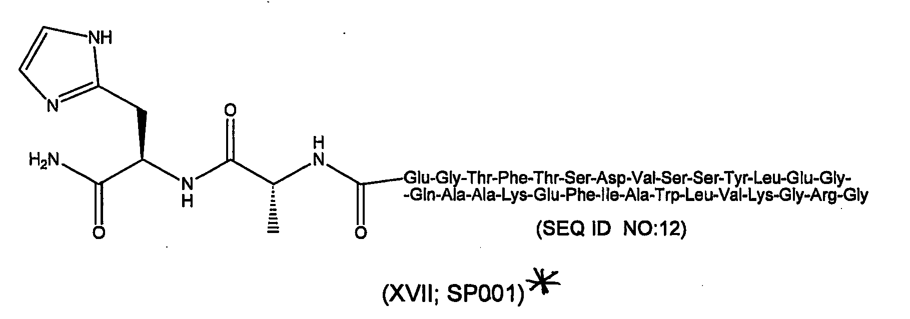 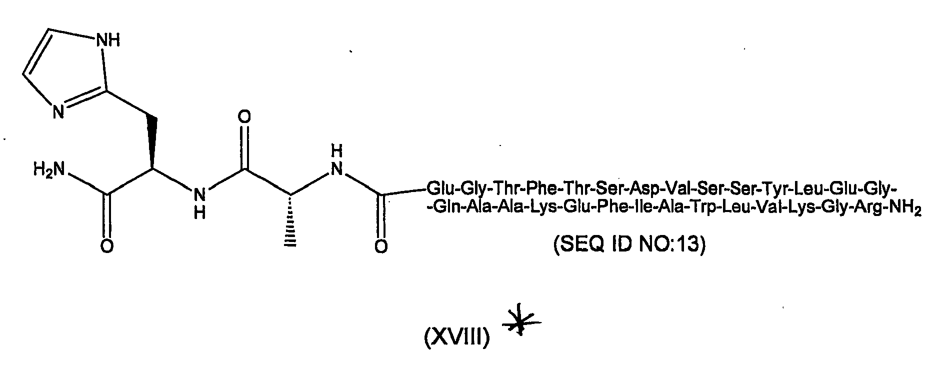 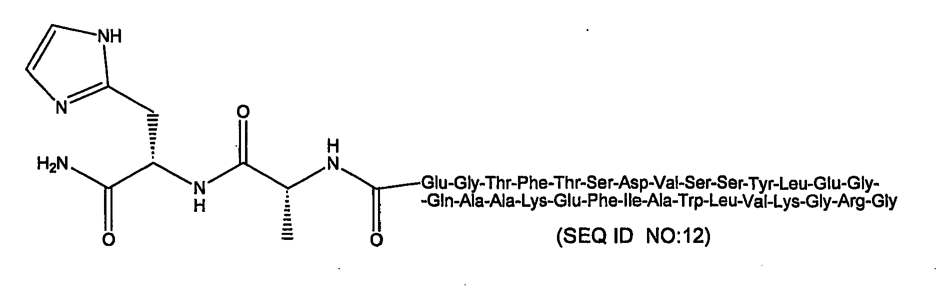 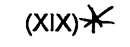 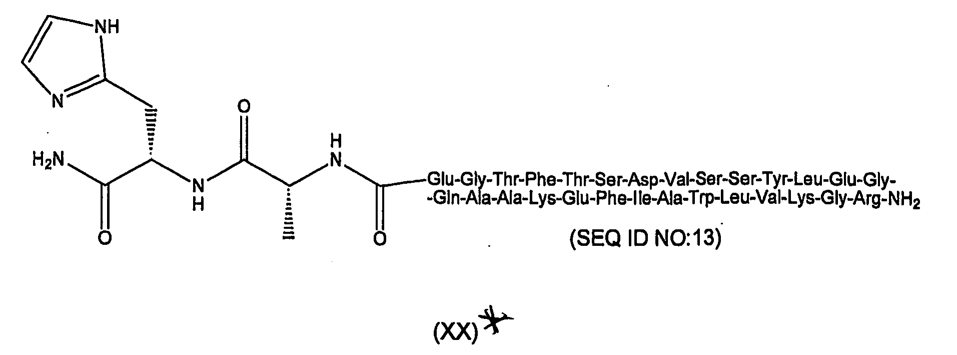 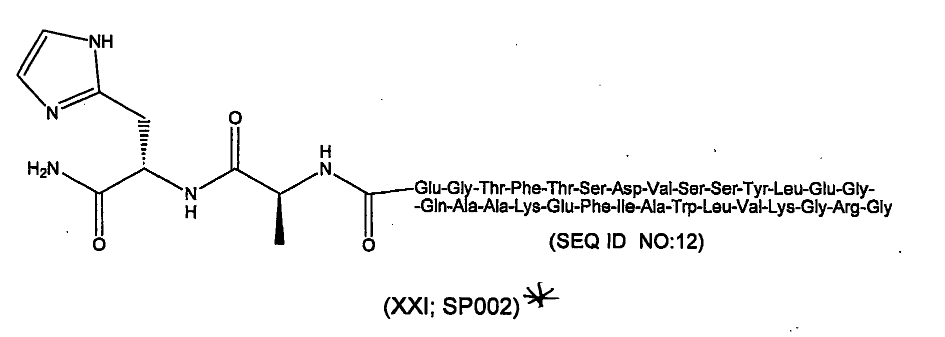 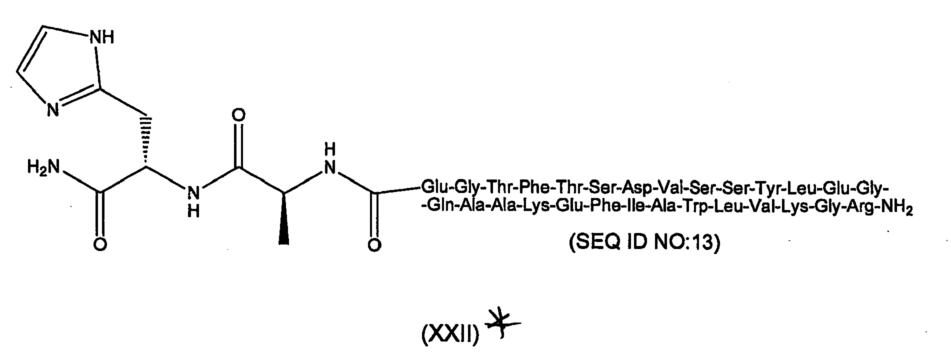 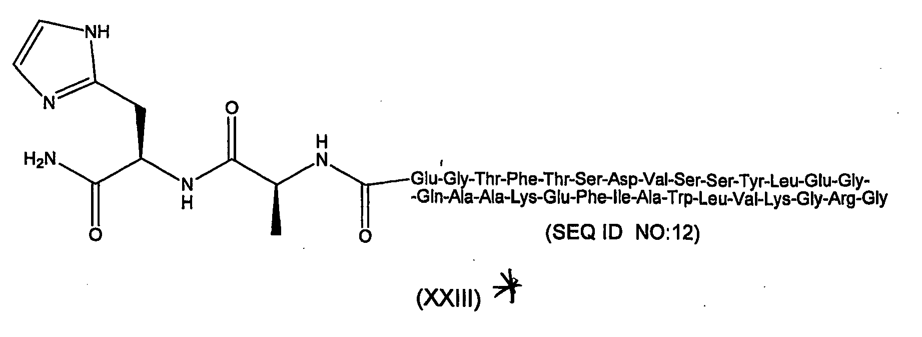 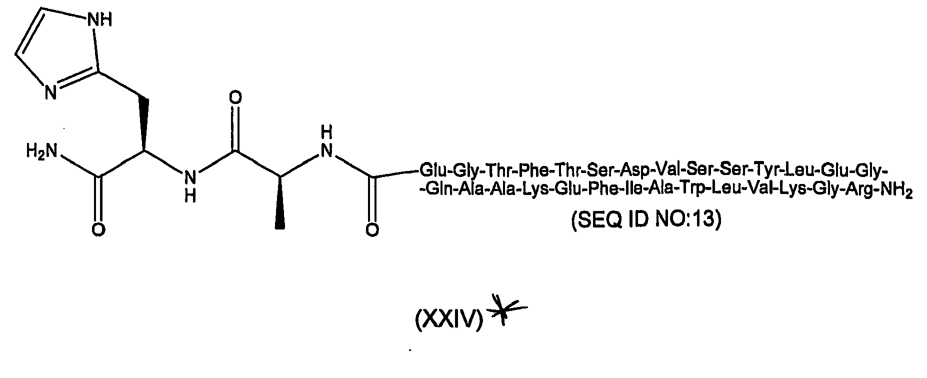 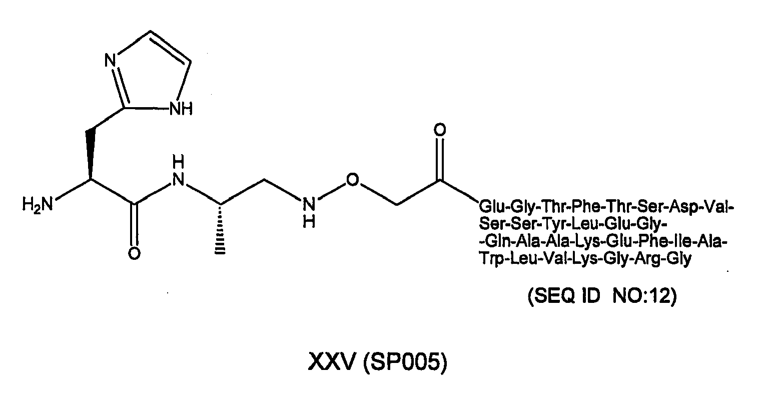 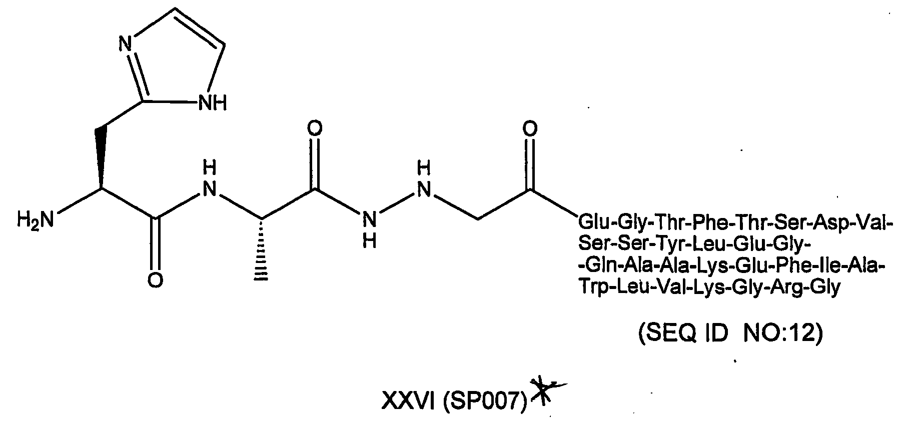 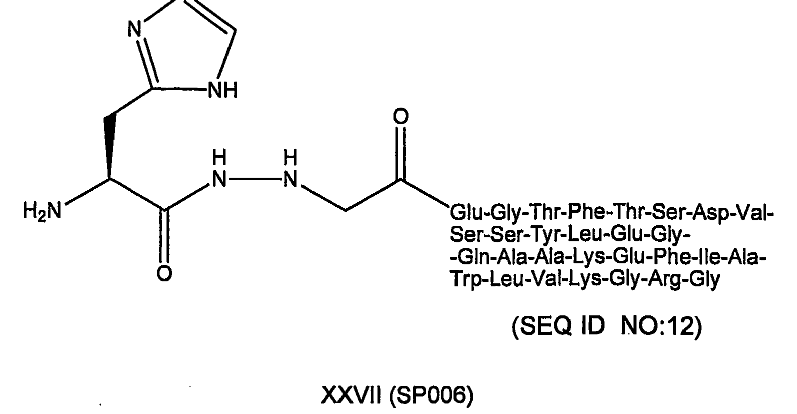 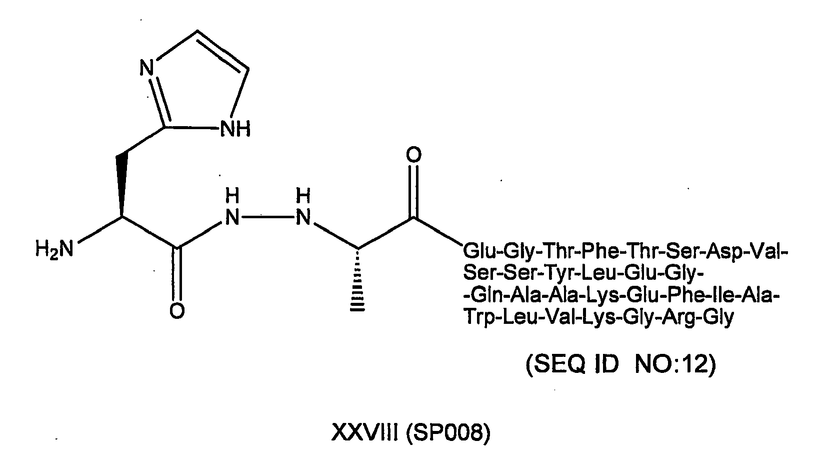 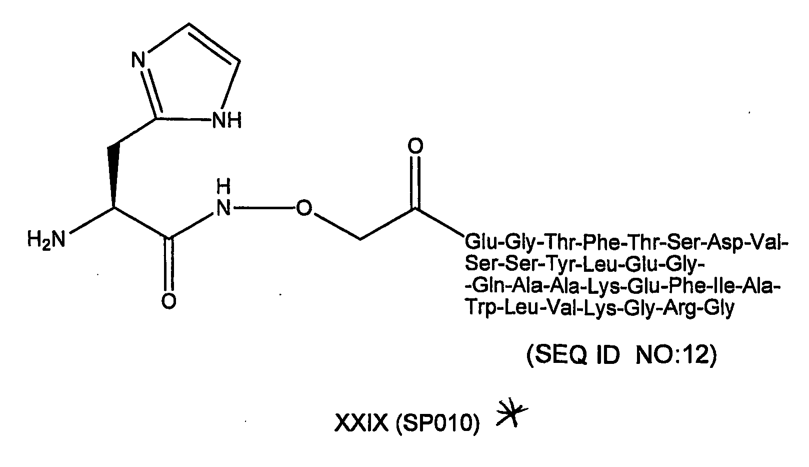 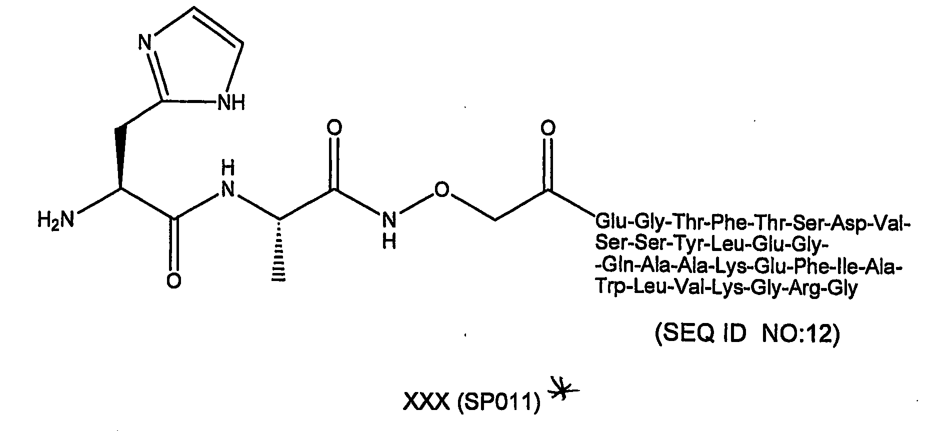 * for illustrative purposes only

### Preparation of Compounds

The invention also encompasses a process for the preparation of the compounds of formula (I) which may be obtained by various methods.

Convenient methods include solid phase sequential synthesis, synthesis and coupling of fragments in solution, enzymatic synthesis, or by employing molecular biology techniques.

The general methods of solid phase peptide synthesis have been described in: "Fmoc Solid Phase Peptide Synthesis: A Practical Approach"; Chan, W.C. and White, P.D. eds., Chapter 3, pp 41-76, Oxford University Press, 2000; "Chemical Approaches to the Synthesis of Peptides and Proteins"; P Lloyd-Williams et al, CRC Press 1997; and "The Chemical Synthesis of Peptides", John Jones, Oxford University Press, 1994.

Solid phase synthesis is for example carried out using an automatic device which executes in a repetitive and programmable manner the deprotection, coupling and washing cycles necessary for the sequential introduction of amino acids into the peptide chain.

The C-terminal amino acid is fixed on a resin conventionally used for the preparation of polypeptides, preferably a polystyrene cross-linked with 0.5 to 3.0% divinylbenzene and provided with activated radicals that enable the first amino acid to be fixed covalently to the resin. Suitable resins are well known to those skilled in the art. Appropriate selection of the resin allows the introduction after synthesis of the C-terminal Z function.

The amino acids are then introduced one by one in the desired sequence. Each cycle of synthesis corresponding to the introduction of an amino acid comprises N-terminal deprotection of the peptide chain, successive washings designed to remove the reagents or swell the resin, coupling with activation of the amino acid and further washings. Each of those operations is followed by filtration effected as a result of the presence of a sintered glass filter incorporated in the reactor in which the synthesis is beings carried out.

The couplings reagents used are the conventional reagents for peptide synthesis, such as dicyclohexylcarbodiimide (DCC) and hydroxybenzotriazole (HOBT) or benzotriazol-1-yl-oxytris(dimethylamino)phosphonium hexafluorophosphate (BOP), or also diphenyl-phosphorylazide (DPPA). Activation by the formation of mixed or symmetrical anhydrides is also possible.

Each amino acid is introduced into the reactor in an approximately 6-fold excess in relation to the degree of substitution of the resin and in an approximately equivalent amount in relation to the coupling agents. The coupling reaction may be confirmed at each stage of the synthesis by the ninhydrin reaction test described by Kaiser et al. (Anal. Biochem., 34, 595, 1970).

After assembling the peptide chain on the resin, an appropriate treatment, for example using a strong acid such as trifluoroacetic acid, or hydrofluoric acid in the presence of anisole, ethanedithiol or 2-methylindole, is used to cleave the peptide from the resin and also to free the peptide of its protecting groups. Alternatively, the group Z may be introduced at this stage, concomitant with cleavage from the resin. Dependent on the nature of the group Z, the skilled person will be able to select suitable conditions for such a transformation.

Alternatively, the peptide may be cleaved from the resin with a free carboxyl group, which may subsequently be coupled to the group Z. Again, the skilled person will be able to determine suitable conditions.

The compound is then optionally purified by conventional purification techniques, especially chromatography techniques.

Alternatively, the peptide may be assembled in the N to C direction. Suitable methodology is described in GB0505200.6, the contents of which are incorporated herein by reference.

The peptides of the present invention may also be obtained by coupling in solution selectively protected peptide fragments which may themselves be prepared either in the solid phase or in solution. The use of protecting groups and the exploitation of their differences in stability is analogous to solid phase methods with the exception of the attachment of the peptide chain to the resin. The C-terminal carboxy group is protected, for example, by a methyl ester or an amide function. The methods of activation during coupling are likewise analogous to those employed in solid phase synthesis.

The peptides of the present invention may also be obtained using molecular biology techniques, employing nucleic acid sequences that encode those peptides. Those sequences may be RNA or DNA and may be associated with control sequences and/or inserted into vectors. The latter are then transfected into host cells, for example bacteria. The preparation of the vectors and their production or expression in a host are carried out by conventional molecular biology and genetic engineering techniques.

The introduction of the linker group Y may be achieved as a step in the solid-phase synthesis of peptides as described above, or alternatively in solution. For example, compounds of formula (VII) above wherein R₈ is O are suitably prepared as shown in scheme (I).

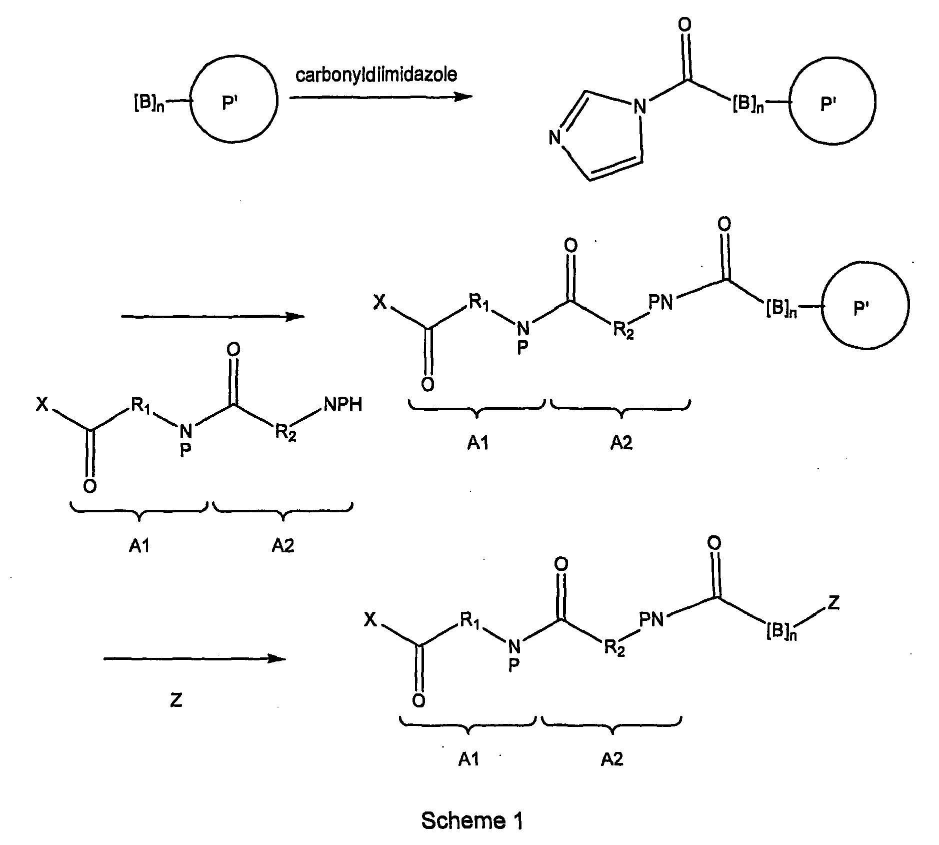

Peptide [B]ₙ is assembled conventionally (as described above) on solid support P' in the C to N direction. Therefore, the C-terminus of [B]ₙ is attached (optionally via a linker) to the solid support, and the N terminus is free. The peptide is reacted with carbonyldiimidazole to give derivative. This is subsequently reacted with dipeptide X-A₁-A₂-NPH to give solid-bound urea. This is cleaved from the solid support (e.g. in the presence of Z) to give the compound of the invention.

### Other forms

The invention also encompasses all pharmaceutically acceptable forms of compounds of formula I, including without limitation, its free form (zwitterion), and its pharmaceutically acceptable complexes, salts, solvates, hydrates, and polymorphs. Salts include, without limitation, acid addition salts and base addition salts, including hemisalts.

Pharmaceutically Acceptable Salts

The present invention also encompasses pharmaceutically acceptable salts of the present compounds. Such salts include pharmaceutically acceptable acid addition salts, pharmaceutically acceptable metal salts, ammonium and alkylated ammonium salts.

Acid addition salts include salts of inorganic acids as well as organic acids. Representative examples of suitable inorganic acids include hydrochloric, hydrobromic, hydriodic, phosphoric, sulfuric, nitric acids and the like.

Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methane-sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like.

Further examples of pharmaceutically acceptable inorganic or organic acid addition salts include the pharmaceutically acceptable salts listed in J. Pharm. Sci. 1977, 66, 2, which is incorporated herein by reference.

Examples of metal salts include lithium, sodium, potassium, magnesium, calcium salts and the like. Examples of ammonium and alkylated ammonium salts include ammonium, methyl-, dimethyl-, trimethyl-, ethyl-, hydroxyethyl-, diethyl-, n-butyl-, sec-butyl-, tert-butyl-, tetramethylammonium salts and the like.

### Methods of Treatment

In one embodiment of the invention a compound according to the invention is used in the treatment or prevention of a condition selected from hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers.

### Combination Therapies

The compounds of the present invention may be combined in use with one or more pharmacologically active substances. Preferred classes of pharmacologically active substances are antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity.

In the present context the expression "antidiabetic agent"includes compounds for the treatment and/or prophylaxis of insulin resistance and diseases wherein insulin resistance is the pathophysiological mechanism.

Examples of these pharmacologically active substances are: Insulin, GLP-1 agonists, sulphonylureas (e. g. tolbutamide, glibenclamide, glipizide and gliclazide), biguanides e. g. metformin, meglitinides, glucosidase inhibitors (e. g. acorbose), glucagon antagonists, DPP-IV (dipeptidylpeptidase-IV) inhibitors, inhibitors of hepatic enzymes involved in stimulation of gluconeogenesis and/or glycogenolysis, glucose uptake modulators, thiazolidinediones such as troglitazone and ciglitazone, compounds modifying the lipid metabolism such as antihyperlipidemic agents as HMG CoA inhibitors (statins), compounds lowering food intake, RXR agonists and agents acting on the ATP-dependent potassium channel of the micelles, e. g. glibenclamide, glipizide, gliclazide and repaglinide; Cholestyramine, colestipol, clofibrate, gemfibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as benazepril, capto pril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine am-phetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, ss3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5_{HT} (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TRss agonists; histamine H₃ antagonists.

It should be understood that any suitable combination of the compounds according to the invention with one or more of the above-mentioned compounds and optionally one or more further pharmacologically active substances are considered to be within the scope of the present invention.

Furthermore, the compound of the invention and the additional therapeutic agent may be present in the same dosage form, or separately for sequential, separate or simultaneous administration.

### Pharmaceutical Compositions

The present invention also provides pharmaceutical compositions comprising a compound of the present invention in combination with a pharmaceutically acceptable carrier, diluent, or excipient. Such pharmaceutical compositions are prepared in a manner well known in the pharmaceutical art, and are administered individually or in combination with other therapeutic agents, preferably via parenteral routes. Especially preferred routes include intramuscular and subcutaneous administration.

Parenteral daily dosages, preferably a single, daily dose, are in the range from about 1 pg/kg to about 1,000 µg/kg of body weight, although lower or higher dosages may be administered. The required dosage will depend upon the severity of the condition of the patient and upon such criteria as the patient's height, weight, sex, age, and medical history.

In making the compositions of the present invention, the active ingredient, which comprises at least one compound of the present invention, is usually mixed with an excipient or diluted by an excipient. When an excipient is used as a diluent, it may be a solid, semi-solid, or liquid material which acts as a vehicle, carrier, or medium for the active ingredient.

In preparing a formulation, it may be necessary to mill the active compound to provide the appropriate particle size prior to combining with the other ingredients. If the active compound is substantially insoluble, it ordinarily is milled to particle size of less than about 200 mesh. If the active compound is substantially water soluble, the particle size is normally adjusted by milling to provide a substantially uniform distribution in the formulation, e.g., about 40 mesh.

Some examples of suitable excipients include lactose, dextrose, sucrose, trehalose, sorbitol, and mannitol. The compositions of the invention can be formulated so as to provide quick, sustained or delayed release of the active ingredient after administration to the patient by employing procedures well known in the art.

The compositions are preferably formulated in a unit dosage form with each dosage normally containing from about 50 µg to about 100 mg, more usually from about 1 mg to about 10 mg of the active ingredient. The term "unit dosage form" refers to physically discrete units suitable as unitary dosages for human subjects and other mammals, each unit containing a predetermined quantity of active material calculated to produce the desired therapeutic effect in association with a suitable pharmaceutical excipient.

Amongst the pharmaceutical compositions according to the invention there are those which are suitable for oral, parenteral or nasal administration, including tablets or dragees, sublingual tablets, sachets, soft gelatin capsules, suppositories, creams, ointments, dermal gels, transdermal devices, aerosols, drinkable and injectable ampoules.

The dosage varies according to the age and weight of the patient, the nature and severity of the disorder and the route of administration.

For the purpose of parenteral administration, compositions containing a compound of the present invention preferably are combined with distilled water and the pH is adjusted to about 6.0 to about 9.0.

Additional pharmaceutical methods may be employed to control the duration of action. Controlled release preparations may be achieved by the use of polymers to complex or absorb a compound of the present invention. The controlled delivery may be exercised by selecting appropriate macromolecules (for example, polyesters, polyamino acids, polyvinylpyrrolidone, ethylenevinyl acetate, methylcellulose, carboxymethylcellulose, and protamine sulfate) and the concentration of macromolecules as well as the methods of incorporation in order to control release.

Another possible method to control the duration of action by controlled release preparations is to incorporate a compound of the present invention into particles of a polymeric material such as polyesters, polyamino acids, hydrogels, poly (lactic acid) or ethylene vinylacetate copolymers.

Alternatively, instead of incorporating a compound into these polymeric particles, it is possible to encapsulate a compound of the present invention in microcapsules prepared, for example, by coacervation techniques or by interfacial polymerization, for example, hydroxymethylcellulose or gelatin-microcapsules, respectively, or in colloidal drug delivery systems, for example, liposomes, albumin microspheres, microemulsions, nanoparticles, and nanocapsules, or in macroemulsions. Such teachings are disclosed in Remington's Pharmaceutical Sciences (1980).

The compounds of the present invention have insulinotropic activity. Thus, another aspect of the present invention provides a method for enhancing the expression of insulin comprising providing to a mammalian pancreatic B-type islet cell an effective amount of a compound of the present invention.

Also intended as pharmaceutically acceptable acid addition salts are the hydrates and other solvates which the present compounds are able to form.

Furthermore, the pharmaceutically acceptable salts comprise basic amino acid salts such as lysine, arginine and ornithine.

The acid addition salts may be obtained as the direct products of compound synthesis. In the alternative, the free base may be dissolved in a suitable solvent containing the appropriate acid, and the salt isolated by evaporating the solvent or otherwise separating the salt and solvent.

The compounds of the present invention may form solvates with standard low molecular weight solvents using methods well known to the person skilled in the art. Such solvates are also contemplated as being within the scope of the present invention.

### Prodrugs

The disclosure also encompasses prodrugs of the present compounds, which on administration undergo chemical conversion by metabolic processes before becoming pharmacologically active substances. In general, such prodrugs will be functional derivatives of the compounds of the general formula (I), which are readily convertible *in vivo* into the required compound of the formula (I). Conventional procedures for the selection and preparation of suitable prodrug derivatives are described, for example, in "Design of Prodrugs", ed. H. Bundgaard, Elsevier, 1985.

For example, it is well known that several forms of GLP-1 are processed *in vivo* from proglucagon; GLP-1 (1-37), GLP-1 (7-37) and GLP-1 (7-36). Accordingly, the person skilled in the art will understand that the present disclosure also embraces peptides incorporating a fragment of structure (I) above which are metabolisable *in vivo* to give compounds of formula (I).

### Definitions

### Multiply Occurring Definitions

Where a term occurs in a formula more than once, it is intended that its use is independent of any other occurrence in that formula; for example, the term N(alkyl)₂ refers to an amine group having two independently selected alkyl substituents.

### Optionally Substituted

Where a group or substituent is itself defined as optionally substituted, unless otherwise specified, it has up to three substituents independently selected from the group consisting of halogen, OH, NH₂, NH(alkyl), N(alkyl)₂, alkyl, alkenyl, alkynyl, alkoxy, carbomoyl, carboxy, cyano, nitro, and SH.

### Alkyl

Alkyl, as used herein refers to an aliphatic hydrocarbon chain and includes straight and branched chains preferably of 1 to 12, more preferably 1 to 6 carbon atoms such as methyl, ethyl, n-propyl, isopropyl, n-butyl, isobutyl, sec-butyl, t-butyl, n-pentyl, isopentyl, neo-pentyl, n-hexyl, and isohexyl.

### Alkylene

The term alkylene as used herein refers to a divalent saturated branched or unbranched hydrocarbon chain containing from 1 to 6 carbon atoms, and includes, for example, methylene (CH₂), ethylene (CH₂CH₂), propylene (CH₂CH₂CH₂), 2-methylpropylene (CH₂CH(CH₃)CH₂), hexylene ((CH₂)₆), and the like.

### Alkenyl

Alkenyl, as used herein, refers to an aliphatic hydrocarbon chain having at least one double bond, and preferably one double bond, and includes straight and branched chains e. g. of 2 to 6 carbon atoms such as ethenyl, propenyl, isopropenyl,but-1-enyl, but-2-enyl, but-3-enyl, 2-methypropenyl.

### Alkynyl

Alkynyl, as used herein, refers to an aliphatic hydrocarbon chain having at least one triple bond, and preferably one triple bond, and includes straight and branched chains e. g. of 2 to 6 carbon atoms such as ethynyl, propynyl, but-1-ynyl, but-2-ynyl and but-3-ynyl.

### Acyl

Acyl as used herein refers to the group -C(=O)alkyl or -C(=O)H, wherein alkyl is as defined above. Examples of acyl groups are formyl (-C(=O)H), acetyl (-C(=O)CH₃), and propanoyl (-C(=O)CH₂CH₃).

### Cycloalkyl

Cycloalkyl, as used herein, refers to a cyclic, saturated hydrocarbon group having from 3 to 8 ring carbon atoms. Examples of cycloalkyl groups are cyclopropyl, cyclobutyl, cyclopentyl, cyclohexyl, cycloheptyl and cyclooctyl.

### Cycloalkylene

The term cycloalkylene as used herein refers to a divalent saturated cyclic hydrocarbon group containing from 3 to 8 ring carbon atoms, and includes, for example, methylene cyclopropylene, cyclobutylene, cyclohexylene and the like.

### Cycloalkylalkyl

The term cycloalkylalkyl as used herein refers to the group -alkylene-cycloalkyl, wherein alkylene and cycloalkyl are as defined above. Examples of cycloalkylalkyl groups include cyclopropylmethyl, 2-cyclobutylethyl, cyclopentylmethyl, cyclohexylmethyl and 4-cycloheptylbutyl, and the like.

### Alkoxy

Alkoxy as used herein refers to the group -O-alkyl, wherein alkyl is as defined above. Examples of alkoxy groups include methoxy, ethoxy, n-propoxy, isopropoxy, n-butoxy, isobutoxy, sec-butoxy, t-butoxy, n-pentoxy, isopentoxy, neo-pentoxy, n-hexyloxy, and isohexyloxy.

### Alkylthio

Alkylthio as used herein refers to the group -S-alkyl, wherein alkyl is as defined above. Examples of alkylthio groups include thiomethyl, thioethyl and thiohexyl.

### Cycloalkoxy

Cycloalkoxy as used herein refers to the group -O-cycloalkyl, wherein cycloalkyl is as defined above. Examples of cycloalkoxy groups are cyclopropoxy, cyclobutoxy, cyclopentoxy, cyclohexyloxy, cycloheptyloxy and cyclooctyloxy.

### Aminoalkyl

Aminoalkyl as used herein refers to the term -alkylene-NH₂, wherein alkylene is as defined above. Examples of aminoalkyl groups include methylamino (-CH₂NH₂) and 2-ethylamino (-CH₂CH₂NH₂).

### Thioalkyl

Thioalkyl as used herein refers to the group -alkylene-SH, wherein alkylene is as defined above. Examples of thioalkyl groups are methylthio (CH₂SH) and 2-ethylthio (CH₂CH₂SH).

### Sulfoxoalkyl

Sulfoxoalkyl as used herein refers to the group -S(O)-alkyl, wherein alkyl is as defined above.

### Sulfonoalkyl

Sulfonoalkyl as used herein refers to a the group -S(O)₂-alkyl wherein alkyl is as defined above.

### Halogen

Halogen, halide or halo-refers to iodine, bromine, chlorine and fluorine.

### Haloalkyl

Haloalkyl as used herein refers to an alkyl group as defined above wherein at least one hydrogen atom has been replaced with a halogen atom as defined above. Examples of haloalkyl groups include chloromethyl, dichloromethyl, trichloromethyl, fluoromethyl, difluoromethyl and trifluoromethyl. Preferred haloalkyl groups are fluoroalkyl groups (i.e. haloalkyl groups containing fluorine as the only halogen). More highly preferred haloalkyl groups are perfluoroalkyl groups, i.e. alkyl groups wherein all the hydrogen atoms are replaced with fluorine atoms.

### Hydroxyalkyl

Hydroxyalkyl, as used herein, refers to an alkyl group as defined above wherein at least one hydrogen atom is replaced by a hydroxyl group. Examples of hydroxyalkyl groups include hydroxymethyl (-CH₂OH), 2-hydroxyethyl (-CH₂CH₂OH), and 1-hydroxyethyl (-CH(OH)CH₃).

### Aryl

As used herein,"aryl" refers to an unsaturated aromatic carbocyclic group of from 6 to 10 carbon atoms having a single ring (e. g., phenyl) or multiple condensed (fused) rings (e.g., naphthyl). Preferred aryl groups include phenyl, naphthyl and the like.

### Heteroaryl

Heteroaryl, as used herein refers to 5 to 10 membered mono or bicyclic aromatic rings having from 1 to 3 heteroatoms selected from N, O and S. Monocyclic rings preferably have 5 or 6 members and bicyclic rings preferably have 8, 9 or 10 membered ring structures. Exemplary heteroaryls include pyrrolyl, furyl, thienyl, imidazolyl, pyrazolyl, oxazolyl, isoxazolyl, thiazolyl, isothiazolyl, pyridyl, pyrazinyl, pyrimidinyl, indolyl, quinolyl and isoquinolyl.

### Alkoxycarbonyl

As used herein, the term alkoxycarbonyl refers to the group -C(=O)-O-Alkyl, wherein alkyl is defined above. Examples of alkoxycarbonyl groups include methoxycarbonyl, ethoxycarbonyl and propoxycarbonyl.

### Aryloxycarbonyl

As used herein, the term aryloxycarbonyl refers to the group -C(=O)-O-Aryl wherein aryl is defined above. Examples of aryloxycarbonyl groups include phenoxycarbonyl and naphthyloxycarbonyl.

### Carboxyalkyl

The term carboxyalkyl as used herein refers to the group -alkylene-CO₂H, wherein alkylene is as defined above.

### Carbamoyl

The term carbamoyl as used herein refers to the group -CONH₂.

### Carbamoylalkyl

The term carbamoylalkyl as used herein refers to the group -alkylene-carbamoyl, wherein alkylene and carbamoyl are as defined above.

### Arylalkyl

Arylalkyl as used herein refers to the group -alkylene-aryl, wherein alkylene and aryl are as defined above. Examples of arylalkyl groups are benzyl (-CH₂Ph) and 2-phenethyl (-CH₂CH₂Ph).

### Arylalkoxy

Arylalkoxy as used herein refers to the group -O-alkylene-aryl, wherein alkylene and aryl are as defined above. Examples of arylalkoxy groups include 2-phenylethoxy (-OCH₂CH₂Ph), 5-phenylpentyloxy (-O-(CH₂)₅Ph) and the like.

### Arylalkoxycarbonyl

Arylalkoxycarbonyl as used herein refers to the group -C(=O)-O-alkylene-aryl, wherein alkylene and aryl are as defined above. Examples of arylalkoxycarbonyl groups are benzyloxycarbonyl (-C(=O)-O-CH₂Ph).

### Heteroarylalkyl

The term heteroarylalkyl as used herein refers to the group -alkylene-heteroaryl, wherein alkylene and heteroaryl are as defined above.

### Guanidinoalkyl

The term guanidinoalkyl as used herein refers to the group -alkylene-NHC(=NH)NH₂ wherein alkylene is as defined above.

### Imidazolylalkyl

The term imidazolylalkyl as used herein refers to the group -alkylene-imidazole, wherein alkylene is as defined above.

### Prodrugs

Also contemplated within the invention are prodrugs, that is compounds capable of undergoing metabolism to give compounds of formula (I) as defined above. Suitable prodrugs are N-oxides and compounds having a quaternary nitrogen.

### Leaving group

The term "leaving group" as used herein refers to any moiety or atom that is susceptible to nucleophilic substitution or elimination. Typically, these are atoms or moieties that when removed by nucleophilic substitution or elimination are stable in anionic form. Examples of leaving groups useful in the present invention include alkyl- or arylsulphonate groups such as tosylate, brosylate, mesylate or nosylate, or halides such as fluoride, chloride, bromide, or iodide.

### Multiply occurring groups

When any variable (e.g. aryl, heterocycle, R⁷ etc.) occurs more than one time in any compound of the invention, its definition at each occurrence is independent of its definition at every other occurrence.

### Examples

### 1. Synthesis of GLP-1 9-37 (SEQ ID NO:6)

The 9-37 sequence was assembled using standard Fmoc methodology as described (Chan, W.C., and White, P. D. in: Fmoc Solid Phase Peptide Synthesis: A Practical Approach. Chan, W.C. and White, P.D. eds., Chapter 3, pp 41-76, Oxford University Press, 2000). Initially, 0.5 g (.2 mmol) of PL-PEGA resin (Polymer Labs) was loaded with the HMPA handle (.8 mmol) using DIPEA and HCTU in DMF for 2 hours until it gave a negative ninhydrin test. The resin bearing this handle (HMPA-PEGA resin) was then coupled to Fmoc-glycine as follows:
1 mmol Fmoc Gly (300mg) was dissolved in 2-3 ml dry DCM, followed by an equal amount of dry THF to fully dissolve the amino acid. 0.75mmol (0.06 ml) of N-methylimidazole was added followed by 1 mmol (297 mg) MSNT. The solution was added under a nitrogen atmosphere to the HMPA-PEGA resin and shaken under nitrogen for 90 minutes. The loaded resin was washed 3 times with DMF, then DCM and checked for amino acid loading by Fmoc release assay. A loading of .357 mmol/g was found. The remainder of the 9-37 sequence was assembled using HCTU catalysed coupling, with recoupling performed if needed to obtain a negative ninhydrin reaction.

### Example 2. Synthesis of NH₂-L-Ala-L-His-carboxamide

RINK amide resin (0.1 mmol, 145 mg, 0.69 mmol/g, Novabiochem) was swollen in DMF for 20 minutes. It was then Fmoc-deprotected with 20% piperidine in DMF for 20 minutes. Fmoc(Trityl)His (0.5 mmol, 310 mg) was coupled to the resin with HCTU and DIPEA for 45 minutes. After deprotection, Fmoc-L-Ala was similarly coupled for 45', and the Fmoc group removed with 20% piperidine. After drying, the His-Ala dimer was cleaved from the resin with 10 ml TFA containing 5% TIS and 5% water for 1 hr. After filtration and precipitation with ether, the L-Ala-L-His-NH₂ gave a satisfactory mass spectrum (MH 225) and was used without further purification. The D-Ala-D-His-NH₂ was synthesised in a similar manner.

### Example 3. Synthesis of GLP-1 NH2-L-His-L-Ala-urea-9-37 (SP001) (SEQ ID NO:6) (for illustrative purposes only)

The GLP-1 9-37 on PEGA resin synthesised above (0.54 g, 0.083 mmol peptide) was Fmoc deprotected with 20% piperidine. It was washed with dry DCM 3 times. A solution of 0.83 mmol (134 mg) of carbonyldiimidazole (CDI) in 4 ml dry DCM containing 0.4 mmol DIPEA (0.066 ml) was added to the resin and the vessel shaken for 1 hour. The resin was washed with DCM, DMF, then dry DCM and the CDI coupling repeated. After washing with DCM, a suspension of the NH₂-L-Ala-L-His-carboxamide dimer (0.4 mmol 90mg) in a mixture of THF:DCM:DMF containing DIPEA (0.85 mmol, 0.14 ml) was added to the resin under nitrogen and the mixture shaken overnight. The resin was then washed with DMF, methanol, methanol/water, methanol, DMF, DCM, then ether. The dried peptide resin was then cleaved with TFA (10 ml) containing by volume TIS(4%), water(4%), 90% phenol solution(2%), and thioanisole (2%) for 1.5 hr. The mixture was filtered into cold ether, the precipitate collected by centrifugation, and washed with cold ether twice more. The white precipitate was dissolved in 6 Molar guanidine hydrochloride and purified by reversed phase HPLC using a Jupiter Proteo 250mm x 10mm column(Phenomenex). The product gave an MH of 3399 (ES-MS) and was isolated in about 10% overall yield.

### Example 4. Synthesis of GLP-1 NH₂-D-His-D-Ala-Urea-9-37 (SP002) (SEQ ID NO:6) (for illustrative purposes only)

In a similar manner to Example 3, the NH₂-D-His-D-Ala-Urea-9-37 analogue was synthesised to give a product of the same mass and analytical HPLC purity.

### Example 5. DPP-IV assay

Both the L7,L8 and D7,D8 analogues of GLP-1 above were subject to dipeptidyl peptidase IV hydrolysis as described in Green, B.D., V.A. Gault, M.H. Mooney, N. Irwin, P. Harriott, B. Greer, C.J. Bailey, F.P.M. O'Harte, and P.R. Flatt. (2004) Degradation, receptor binding, insulin-secreting, and antihyperglycaemic actions of palmitate-derivatised native and Ala8-substituted GLP-1 analogues. Biological Chemistry. 385, pp 169-177.

Briefly, 100 - 200 microgams of each peptide was dissolved in 0.5 ml of 50 mM triethanolamine buffer, pH 7.8. To this was added a 10 microlitre solution of 11 milliunits DPP-IV (Sigma) and the solutions incubated at 37°C for up to 42 hours. A control peptide, GLP-1 7-22, was treated identically with DPP-IV. The incubations were terminated by addition of 5 microlitres TFA to each tube and 50 microlitre of each were injected onto a reversed phase 4.6 x 250 mm C₁₈ HPLC column, eluting a gradient of 20% - 45% acetonitrile in 0.1 % TFA/water over 25 minutes. After 18 hr incubation, the N terminal dipeptide of the GLP-1 7-22 sequence was completely cleaved to give the 9-22 sequence (MH-1491) as seen by HPLC and ES-MS, while the 2 GLP-1 analogues of the invention remained intact under these conditions. After 42 hours, the 2 analogues slowly degraded to a product that gave a mass spectrum consistent with the mass of the 9-37 sequence, indicating longer term instability of the urea bond. This represented as much as 15% of the product by HPLC. This degradation appeared to be non-DPP-IV related, since samples without enzyme gave the same profile.

### Example 6 Synthesis of

Example: This analogue features a reduced peptide bond (ψ) (bold) derived from the condensation of Fmoc-alanine aldehyde to an aminooxy-acetyl linker.

### Preparation of Fmoc-amino-oxyacetic acid.

Boc-amino-oxyacetic acid (1 g, 5.23 mmol, Merck Novabiochem) was deprotected with 30% TFA in DCM (5 ml) for 1 hr. to remove the Boc group. After evaporation, amino-oxyacetic acid was dissolved in 25 ml of 10% aqueous potassium carbonate and cooled on ice. N-(9-Fluorenylmethoxycarbonyl)-succinimide (Fmoc-OSu) (2.09 g, 6.23 mmol) was dissolved in 18 ml dioxane/THF (2:1) and added dropwise over 10 minutes to the cooled solution with stirring. A few ml additional THF were added when cloudiness developed and the mixture stirred at room temperature overnight. H₂O (100 ml) was added to the mixture and stirred 10 min to give a cloudy solution. The aqueous phase was extracted once with ethyl ether (50 ml), then ethyl acetate (50 ml), and cooled on ice. The pH was brought to 2.3 with 6N HCl and the resultant cloudy solution extracted with ethyl acetate (4 x 70 ml). The ethyl acetate phase was backwashed with 0.1N HCL (1 x 50 ml), H₂O (1 x 50 ml), and saturated sodium chloride (1 x 40 ml) before being dried over anhydrous sodium sulfate. TLC on silica gel G60 plates developed in chloroform:methanol:acetic acid (8:1:1) showed the product as a UV positive spot with an R_{F} of about 0.5. HPLC of the crude material on a reversed-phase Jupiter analytical C4 column in a 0.1% TFA/H₂O/acetonitrile gradient and gave a peak at 21.2 min. showing a mass of 314.2 (MH+) by electrospray MS in 77% purity, but containing excess N-hydroxysuccinimide (NHS). This was removed by evaporating the ethyl acetate and re-dissolving the white amorphous solid in 150 ml ethyl ether. A white, granular precipitate developed which was filtered out. The ether phase was concentrated to 40 ml and hexane (200 ml) was added to precipitate the N-Fmoc amino-oxyacetic acid at 4° overnight. Filtration gave the product (1^{st} crop, 1.08 g, 3.46 mmol) in 84% purity by HPLC. A second crop of 50 mg resulted in an overall yield of 1.13g (3.6 mmol, 69%).

### Preparation of Fmoc-alanyl aldehyde

This building block was more easily obtained via the initial preparation of the Boc-alanine diol intermediate as described below:

### Preparation of mixed anhydride of N-Boc-alanine and isobutyl carbonate

N-Boc Alanine (4.05 g, 21.42 mmol) was dissolved in 200 ml dry THF and cooled on ice. Redistilled triethylamine( TEA, 3.28 ml, 23.5 mmol) was added followed by the dropwise addition of isobutyl chloroformate (21.5 mmol, 2.84 ml) over 15 min.

Solution was stirred on ice for 2.5 hr and filtered to remove the triethylammonium chloride.

A 250 ml diazomethane solution in DCM was prepared from 12 g N-nitrosomethylurea by treatment with 20 ml of 50%(w/w) potassium hydroxide solution in dry DCM at 4° by a standard method (F. Arndt, Org. Synth., 1943, Coll. Vol. 2, p. 165).

The filtrate containing the mixed anhydride was concentrated to a volume of 60-70 ml and added dropwise to the dry diazomethane solution on ice over a 15 min period, with evolution of gas. After stirring 1.5 hr at room temp., 10 ml H₂O and about 2 ml acetic acid were added dropwise to decompose excess diazomethane. The aqueous layer was separated, and the yellow organic phase was washed quickly with H₂O (2 x 50 ml), .5M sodium bicarbonate(1 x 50 ml), H₂O (1 x 50 ml), then saturated sodium chloride solution (1 x 40 ml). The organic layer containing the N-Boc alanyldiazomethane was dried over sodium sulfate overnight. ES-MS showed the major component to be the diazomethane derivative (MH+ = 214). The organic phase was filtered to remove any precipitated solid, and the solvent evaporated under reduced pressure to give a yellow oil. This was dissolved in 100-125 ml of ether, filtered, and the ether evaporated to give a yellow solid which was dried under high vacuum. Yield was 4.96 g (100%). The N-Boc-alanyl diazomethane was converted to N-Boc-alanylbromomethylketone derivative by dissolving in 30 ml acetone and chilling to 4°. A 48% aqueous solution of HBr (23 mmol, 2.6 ml) was added dropwise, with evolution of gas (N₂). After 10 min, 150 ml chloroform and 10 ml H2O were added, the solution transferred to a separatory funnel and quickly washed with .5M sodium bicarbonate (50 ml), then water. The chloroform layer was dried over sodium sulfate for 1 hr. ES-MS of crude material gave correct doublet masses for the bromo derivative (MH+ = 266, 268), reflecting the natural abundance of the 79 and 81 dalton isotopes of bromine.

### N-Boc-alanyl-alpha keto ester

The bromomethyl ketone derivative was converted to the Boc-alanyl alpha-ketoester using potassium acetate. After evaporation of chloroform, the resulting oil was dissolved in 20 ml DMF and solid potassium acetate (5.6 g, 57.7 mmol) was added with stirring. The mixture was stirred for 5 hr, after which ES-MS showed the disappearance of the bromo derivative and the appearance of the alpha keto ester at MH+ 246. Add 20 ml H₂O and extract aqueous phase with chloroform (2 x 100 ml). Wash chloroform layer with H₂O (30 ml), brine (30 ml), and dry overnight over sodium sulfate. After evaporation of chloroform, the oily product was placed under high vacuum overnight.

### Crude yield -4.114g (18 mmol, 78%)

A portion of this was converted to the Boc-alanine diol by dissolving 2.45g (10 mmol) of the crude alpha keto ester oil in 40 ml of methanol:isopropanol: H₂O (8:1:1), and adding a 2.5 molar excess sodium borohydride (25 mmol, 950 mg) in portions. After 3.5 hr, ES-MS showed the complete conversion to the diol (MH+ 206). The mixture was cooled on ice, 10 ml H₂O was added, and solid citric acid was added with stirring to drop the pH to 5.8. Ethyl acetate (15-20 ml) and H₂O (5 ml) were added followed by 3g of solid sodium chloride. The mixture was then extracted with ethyl acetate (2 x 75 ml) and the organic phase dried over sodium sulfate. The solvent was evaporated, and the oil containing N-Boc-alanine diol placed under high vacuum for several hours. It was then dissolved in about 50 ml ether and stirred, with a small amount of white precipitate forming which was removed by centrifugation. Evaporation of the ether gave 1.39g (6.85 mmol) of a colourless oil (68% yield). The Boc group was removed by treating the oil with 25% TFA in DCM (10 ml) for 35 min. The solvents were evaporated and the oil left under high vacuum overnight to give 1.86 g crude product. ES-MS shows alanine-diol at MH+ 105.9.

This was converted directly to the Fmoc-alanine-diol by dissolving the oil in 10% sodium carbonate (20 ml) and acetone (5 ml) on ice. A few ml dioxane were added to improve solubility. Fmoc-OSu (2.86 g, 8 mmol) was dissolved in 10 ml dioxane:3 ml acetone and added to the alanyl diol on ice. A milky precipitate forms and THF was added to the mixture to a total volume of 100 ml. After overnight stirring at 4°, H₂O (25 ml), and ether (100 ml) were added to give 2 phases. The aqueous phase was extracted once more with 50 ml ethyl acetate, and the combined organic layers washed with H₂O, 0.1 N HCl, H₂O, and saturated sodium chloride solution. The solvent was evaporated to give 4 g of an oily white solid. TLC showed excess Fmoc-OSu and fluorine-containing spots. These were removed by dissolving the solid in warm DCM (80 ml) and leaving at -20° overnight. The white precipitate was filtered, and HPLC showed the N-Fmoc-alanine diol to be 93% pure.

Yield -1.27g (3.9 mmol, 56%) ES-MS showed MH+ at 328.2

### N-Fmoc-alanyl aldehyde

Fmoc-alanine diol (155 mg, .47 mmol) was dissolved in 4.5 ml methanol. A 5-fold excess of sodium periodate (0.5g, 2.3 mmol) was added and the mixture stirred 3 hr. TLC (silica gel 60 plates) showed complete disappearance of the diol, with aldehyde appearing at R_{F} .6 - .7 (diethyl ether developing solvent). The solvent was evaporated, and the crude aldehyde suspended in ether (10 ml) and filtered . The aldehyde was purified by loading of the ether solution on a 17 x 2.5 cm silica gel column, with diethyl ether as eluting solvent, collecting 12-15 ml fractions. The aldehyde appeared in fractions 1-6, the solvent evaporated, and the recovered Fmoc alanyl aldehyde (95 mg, 68%) kept at -20° until use. ES-MS showed the correct MH+ at 296.2 mu.

### Synthesis of SP005

GLP-1 9-37 was assembled by Fmoc solid phase chemistry as for the other analogues. Following Fmoc deprotection of Glu9, 45 mg of Fmoc-aminooxyacetic acid prepared as above was coupled to 300 mg of resin-bound 9-37 by HCTU activation for 70 minutes. The coupling was repeated with 50 mg of Fmoc-aminooxyacetic acid for 1 hr. until ninhydrin analysis of the resin was negative. Following Fmoc deprotection, the resin was double coupled with 0.4 mmol Fmoc-alanyl-aldehyde (118 mg) in 4 ml DCM:DMF (1:1) for 3 hr each. Ninhydrin analysis of the resin was negative. The resin-bound imine was reduced by treatment with 110 mg (1.74 mmol) sodium cyanoborohydride in 4 ml DMF:DCM:acetic acid (5:4:1) for 2 hr. After washing with DMF, the reduction was repeated for a further 2 hr. The Fmoc group was removed and Fmoc-His was coupled in the usual manner with HCTU until a negative ninhydrin test was obtained. Following final Fmoc deprotection, the completed peptide was cleaved from the resin with TFA containing 4% triisopropylsilane(TIS)/4% H₂O/2%of a 90% phenol solution for 1.5 hr. After ether precipitation and washing, the peptide was purified by HPLC to give a product showing a mass of 3414 by ES-MS.

### Example 7: Synthesis of L-His-NH-NH-CH2-C(O)-Glu9-37 SP006

This analogue features a hydrazide-acetate linkage (bold) between Histidine 7 and Glutamic acid 9. This linkage is a glycine analogue (N-amino glycine), and was assembled by first coupling 2-bromoacetic acid to the N terminus of GLP-1 9-37 resin, followed by displacement of the bromo group with Fmoc-hydrazide, and by final coupling of Histidine 7.

### Preparation of Fmoc-hydrazide

Tert-butyl carbazate (Sigma-Aldrich, 1.32 g, 10 mmol), was dissolved in 10 ml H₂O. After addition of 10 mmol TEA (1.4 ml), a 15 ml solution of 10 mmol Fmoc-OSu (3.37g) in acetonitrile was added dropwise over 10 min. The pH dropped to 8.4, and was maintained at 8.5-9.0 by addition of TEA. After 30 additional min, the solution was filtered, and the filtrate added with stirring to a 100 ml aqueous solution of 20% citric acid. An oily white precipitate resulted. This was extracted with 2 x 150 ml portions of ethyl acetate. The ethyl acetate solution was washed with H₂O (50 ml), saturated sodium chloride, then dried over sodium sulfate. The ethyl acetate volume was reduced, and petroleum ether was added to precipitate the product at 4o overnight. After filtration and drying, 1.95 g (5.4 mmol, 54%) was recovered. ES-MS showed the presence of excess NHS. The crude product was re-dissolved in 100 ml ethyl acetate, a fine white precipitate was filtered out, and the ethyl acetate solution washed with H₂O (2x) and brine (1 x). After drying over sodium sulfate and evaporation, 1.64 g (4.6 mmol, 46%) of Fmoc-NH-NH-Boc was recovered which was free of NHS. This was treated with 15 ml of 23% TFA in DCM for 45-50 min to remove the Boc group. The resulting orange solution was evaporated, triturated with DCM, evaporated, and finally evaporated from ethyl acetate. The residue was dissolved in 125 ml ethyl acetate and washed with 10% sodium bicarbonate (2 x 50 ml), H₂O (50 ml), then brine (40 ml). After drying over sodium sulfate, the product was recrystallized from chloroform:petroleum ether overnight at 4°. Yield - 0.95 g (3.74 mmol, 37% from starting Boc-hydrazide) Purity by HPLC (at 290nm) was >96%.
ES-MS gave MH+ 255.1

### Synthesis of SP006

GLP-1 9-37 was assembled by Fmoc solid phase chemistry as for the other analogues. Following Fmoc deprotection of Glu9 on 0.2 mmol peptidyl resin, a solution of 2 mmol 2-bromoacetic acid (278 mg) and 1 mmol DCC (206 mg) in 4 ml DCM:DMF (1:1) were added and shaken for 1 hr. The resin was washed with DMF and DCM, and the resulting ninhydrin test was usually negative. To 50 micromoles bromoacetylated resin was added a solution of 0.5 mmol Fmoc -hydrazide (127 mg, 10 x excess), 0.25 mmol DIPEA (0.04 ml) in 3 ml DMF and the mixture shaken overnight. After washing with DMF, the completeness of the coupling was assessed by measuring the release of the Fmoc group from the terminal Fmoc-hydrazide of a few mg of resin. This was found to be 39% of theoretical, and the coupling was repeated. A final treatment with 0.04 ml hydrazine in DMF for 3 hr was performed to drive the displacement to completion. Following Fmoc deprotection with 20% piperidine, Fmoc-His was double-coupled to the resin until it gave a negative ninhydrin.

The completed peptide was cleaved and deprotected using TFA containing 4% TIS/5% H₂O/2.5% Phenol / 2.5% thioanisole for 1 hr 45 min. After ether precipitation and washing, the peptide was purified by RP-HPLC. ES-MS of purified material gave a mass of 3357.

### Example 8: Synthesis of L-His-L-Ala-NH-NH-CH2-C(O)-Glu9-37 SP007 (for illustrative purposes only)

This analogue also bears the hydrazine linkage (bold) but contains an additional alanine compared to SP006. Fmoc hydrazine was coupled to a 50 micromole portion of bromoacetylated resin from the SP006 synthesis. Following Fmoc deprotection, Fmoc-alanine and Fmoc Histidine were coupled in succession to complete the sequence. The peptide was cleaved from the resin and HPLC purified as for SP006.
ES-MS gave a mass of 3428

### Example 9: Synthesis of L-His-NH-NH-CH(CH3)-C(O)-Glu9-37 SP008 L-Ala

GLP-1 9-37 was assembled by Fmoc solid phase chemistry as for the other analogues. Following Fmoc deprotection of Glu9 on 0.2 mmol peptidyl resin, a solution of 3 mmol of R(+)-2-bromopropionic acid (271 microlitre) and 3 mmol diisopropylcarbodiimide (DIC) (469 microlitres) in 5 ml DCM:DMF (1:1) was added and the slurry shaken for 2 hr. After washing, ninhydrin was positive and the coupling was repeated once. Fmoc-hydrazide (1 mmol, 254 mg) and DIEA (1.18 mmol, .195 ml) in 5 ml DMF were added and the coupling allowed to proceed for 6 hr at 37o, then at room temp for 60 hr. Fmoc release of the resin showed a failed coupling, so the resin was treated with a 4 ml DMF solution of 0.07 ml hydrazine and 11 ml DIPEA for 2.5 hr. After washing with DMF, a few mg of resin was test cleaved with TFA and scavengers to determine if the hydrazine coupling worked. HPLC showed the hydrazide derivative of the GLP-1 resin to be the major species present (ES-MS M=3234.4), and so the synthesis was completed with the coupling of Histidine 7, which gave a negative ninhydrin after 1 hr. The peptide was Fmoc-deprotected and cleaved from the resin with TFA containing 5%H₂O/4%TIS/2.5% phenol/2% thioanisole for 1.5 hr. Following ether precipitation and washing, it was HPLC purified and gave a mass of 3371 by ES-MS.

### Example 10: Dipeptidyl peptidase IV (DPP-IV) assays

GLP-1 analogues were subjected to DPP-IV digestion at pH 7.5 at 37°C. Their stabilities were monitored by RP-HPLC, noting the disappearance of the intact sequence and appearance of a species missing the N terminal dipeptide or similar fragment. Shown below is the percentage of each analogue remaining after the indicated time of incubation on the left.

| | | PBS/TEthA, pH 7.5, 37°, DPP-IV | | | |
|---|---|---|---|---|---|
| Time(hr) | GLP-1 | SP005 | SP006 | SP007 | SP008 |
| 0 | 95 | 95 | 95 | 95 | 95 |
| 22 | <5 | 95 | 88 | <5 | 95 |
| 47 | <5 | 93 | 87 | <5 | 93 |

The native GLP-1 was completely hydrolyzed within 3 hr. It can be seen that only the SP007 is susceptible to DPP-IV hydrolysis, more than 90% hydrolyzed within 22 hr., the major new species being a peak at 3220 mu, indicating a loss of the His-Ala dimer.

In the control study, peptides were incubated with PBS/triethanolamine solution in the absence of DPP-IV to measure their chemical stability.

| | | PBS/TEthA, pH 7.5, 37°, no DPP-IV | | | |
|---|---|---|---|---|---|
| Time (hr) | GLP-1 | SP005 | SP006 | SP007 | SP008 |
| 0 | 98 | 98 | 98 | 98 | 98 |
| 22 | 98 | 95 | 88 | 95 | 91 |
| 47 | 98 | 89 | 88 | 95 | 77 |

Only the SP008 derivative showed significant non-enzymatic degradation (20-25%) after 47 hr under these conditions by HPLC.

### Example 11: Stimulation of Insulin Release

The GLP-1 analogues were evaluated for their ability to stimulate insulin release in a beta TC-6 cell line. This was derived from a pancreatic tumor (insulinoma) arising in a transgenic mouse.

### Cell culture

Beta-TC-6 cells were purchased from A.T.C.C. (A.T.C.C. number CCL-11506; Manassas, VA, U.S.A.). Beta-TC-6 cells were cultured in Dulbecco's Modified Eagle's medium supplemented with 15% (v/v) heat-inactivated foetal bovine serum, 1% (v/v) glutamine, 0.45% (w/v) glucose and 0.15% (w/v) sodium bicarbonate at 37 °C in a humidified 5% CO₂ atmosphere. Beta-TC-6 cells form islands and the monolayer never becomes confluent; cells were passaged at 70% confluence (7 days from seeding) at a subcultivation ratio of 1:4. The monolayer was disrupted from the surface of the culture flask with 0.25% (w/v) Trypsin-0.53mM EDTA.

### Insulin release assay

Beta-TC-6 cells were cultured in 12 well plates for between 4-8 days prior to use. Cells were cultured in Krebs-Ringer (10 mM Hepes, pH 7.4, 136 mM NaCl, 4.7 mM KCl and 1.25 mM MgSO₄) supplemented with 5mM glucose for 1 hr at 37 °C in a humidified 5% CO₂ atmosphere. This medium was removed, cells washed with Krebs-Ringer. Beta-TC-6 cells were then cultured in 1ml per well of Krebs-Ringer supplemented with 5mM glucose and 0, 0.12, 0.6, 3 and 15µM of peptide in duplicate for 30 minutes at 37°C in a humidified 5% CO₂ atmosphere. Medium was removed, diluted 1:5, and insulin concentration determined using an insulin ELISA kit (Mercodia, Uppsala, Sweden) according to the manufacturer's protocol. Beta-TC-6 cells were washed three times with ice-cold PBS and extracted into 0.5ml lysis buffer (62.5 mM Tris, pH 7.5, and 1%, v/v, SDS) per well. Protein concentration was determined by Lowry assay. Insulin release was expressed both as ng insulin released/mg total protein in well/minute and as a percentage, with the value of ng insulin released/mg protein/min for 0µM peptide taken as 100%. *p* values were determined using a Fisher's Exact T test at 95% confidence interval. Only single-sided *p* values are shown.

The results in the table below show insulin release in response to both 15µM and 3 µM GLP-1 analogues. Native GLP-1 7-37 and Exendin-4 were used as positive controls, and GLP-1 9-37 used as a negative control. The values of insulin release for cells only varied from 1.37 - 3.67 ng/min/mg, and depended on "split" number, when aliquots of cells were taken from the main culture flask and aliquoted into individual wells for specific experiments.

| Peptide | Insulin release (ng/min/mg) | | | | | |
|---|---|---|---|---|---|---|
| | 15µM | SD | *p*(single-sided) | 3µM | SD | *p* |
| Native GLP-1 7-37 | **5.56** | **± 2.24** | **.019** | 3.40 | ±1.65 | .3047 |
| SP001 (L-Urea) | 1.83 | ± 1.20 | ND | 1.59 | ±0.59 | ND |
| SP002 (D-Urea) | **3.52** | **± 1.83** | **.0104** | **2.58** | **±0.24** | **.0045** |
| SP005 (reduced oxime) | **5.26** | **± 1.41** | **.0288** | **2.25** | **±0.48** | **.0159** |
| SP006 (His-hydrazine-Gly) | **8.88** | **± 3.02** | **.0483** | **3.86** | **±1.66** | **.1623** |
| SP007 (His-Ala-hydrazine-) | **8.08** | **± 3.90** | **.0572** | 4.51 | ±3.05 | ND |
| SP008 (His-hydrazine-Ala) | **6.20** | **±1.41** | **.0662** | 4.35 | ±1.96 | ND |
| Native GLP-1 9-37 control | 3.25 | ±0.95 | ND | | | |
| Exendin-4 (Exenatide) | 3.09 | ±0.93 | ND | | | |
| Cells only | 1.37 | - 3.67 | | | | |

| | | | | | | |
|---|---|---|---|---|---|---|
| ND = not determined Values considered significant are in bold. | | | | | | |

The table below expresses the values for insulin release for each analogue at 15 µM as a percentage of insulin release by cells only (cells alone = 100%).

| Peptide SP number | Activity of 15 µM of each peptide as percentage of insulin release bv cells only |
|---|---|
| Native GLP-1 7-37 | 196 ± 36 |
| SP001 (L-Urea) | 100 ± 47 |
| SP002 (D-Urea) | **254 ± 70** |
| SP005 | **358 ± 128** |
| SP006 | **330 ± 89** |
| SP007 | **235 ± 91** |
| SP008 | **170 ± 48** |
| Native GLP-1 9-37 control | 123 ± 17 |
| Exendin-4 (Exenatide) | 172 ± 57 |

The table below expresses the values for insulin release for each analogue at 3 µM as a percentage of insulin release by cells only (cells alone = 100%).

| **Peptide SP number** | Activity of 3 µM of each peptide as percentage of insulin release bv cells only |
|---|---|
| Native GLP-1 7-37 | 110 ± 20 |
| SP001 (L-Urea) | 92 ± 28 |
| SP002 (D-Urea) | **192 ± 38** |
| SP005 (reduced oxime) | **171 ± 53** |
| SP006 (His-hydrazine-Gly) | **154 ±25** |
| SP007 (His-Ala-hydrazine-) | 113 ± 65 |
| SP008 (His-hydrazine-Ala) | 112 ± 44 |
| Native GLP-1 9-37 control | ND |
| Exendin-4 (Exenatide) | ND |

### Abbreviations

GLP-1, glucagon-like peptide-1; DPP-IV, dipeptidyl-peptidase; mPEG, monomethoxy polyethylene glycol; CDI, carbonyldiimidazole; DMF, dimethylformamide; DIPEA, diisopropylethylamine; HMPA, 2-(4-hydroxymethyl phenoxy)acetic acid; DCM, dichloromethane; THF, tetrahydrofuran; Trityl, triphenylmethyl; MSNT, 1-(mesitylene-2-sulphonyl)-3-nitro-1*H*-1,2,4-triazole; HCTU, O-(1H-6-Chlorobenzotriazole-1-yl)-1,1,3,3-tetramethyluroniumhexaflourphosphate; TFA, trifluoroacetic acid; TIS, triisopropylsilane.

## Claims

1. A compound of formula (XXV), (XXVII) or (XXVIII): 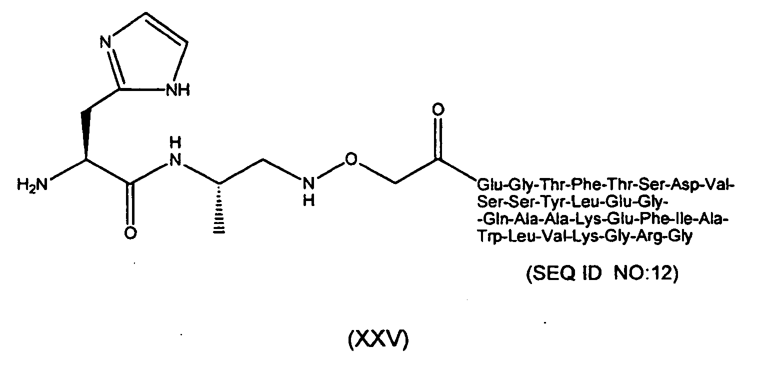 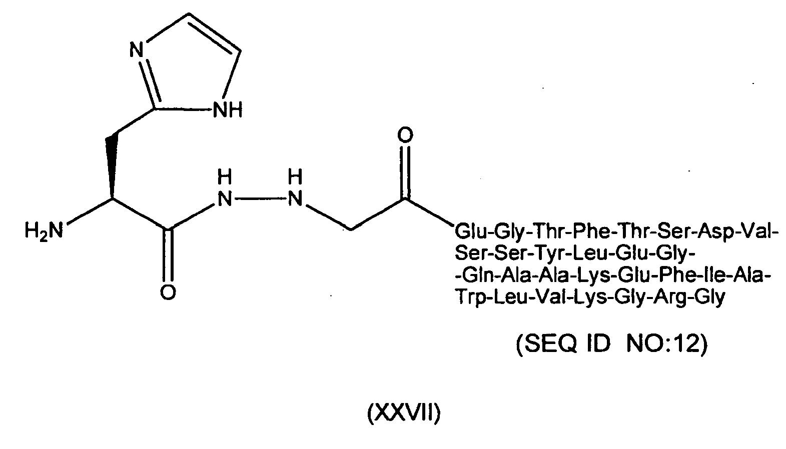 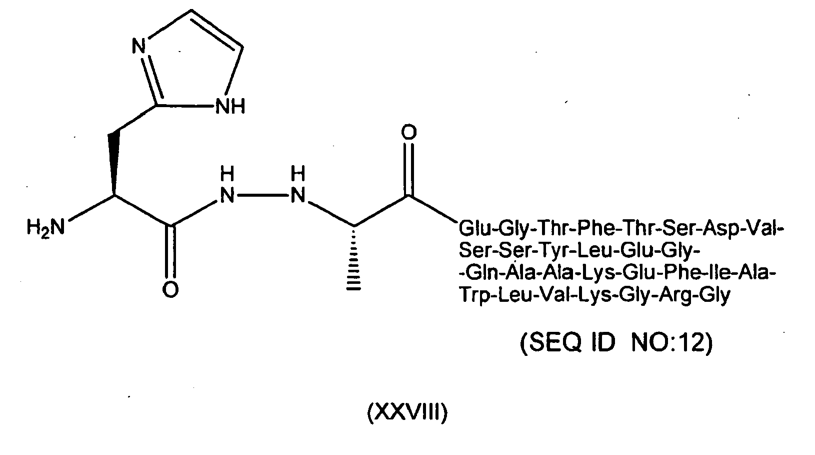 or a pharmaceutically acceptable salt form thereof.

2. A compound as claimed in claim 1 for use as a medicament.

3. A pharmaceutical composition comprising a compound as claimed in claim 1 and a pharmaceutically acceptable carrier or excipient.

4. A compound as claimed in claim 1 for use in the treatment of a mammal suffering from a condition selected from hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers.

5. Use of a compound as claimed in claim 1 in the preparation of a medicament for the treatment of a condition selected from hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers.

## Patentansprüche

1. Verbindung der Formel (XXV), (XXVII) oder (XXVIII): 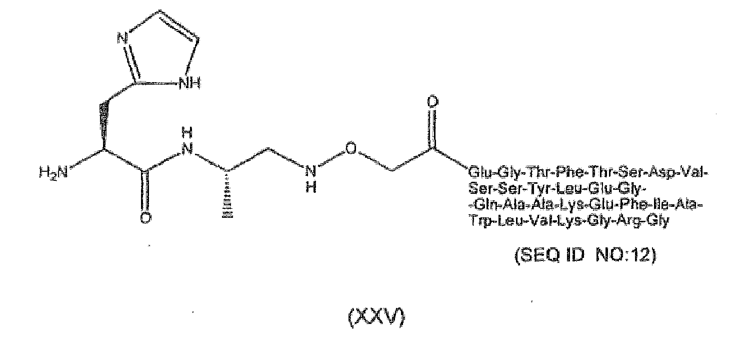 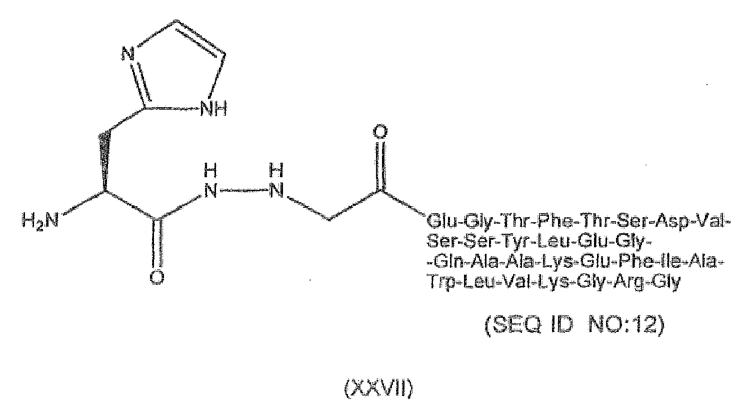 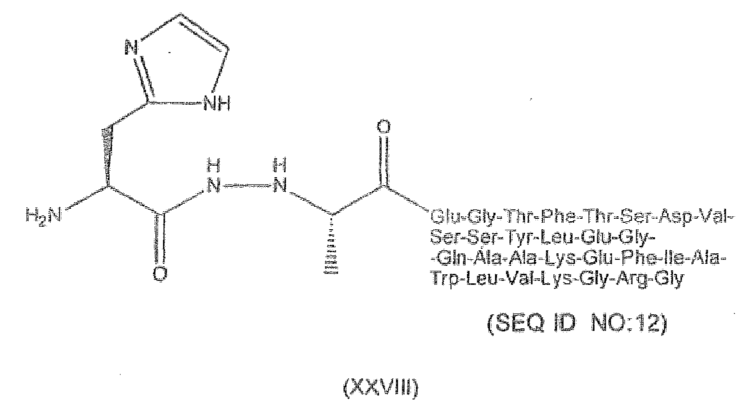 oder eine pharmazeutisch verträgliche Salzform davon.

2. Verbindung nach Anspruch 1 für die Verwendung als ein Arzneimittel.

3. Pharmazeutische Zusammensetzung umfassend eine Verbindung nach Anspruch 1 und einen pharmazeutisch verträglichen Träger oder Hilfsstoff.

4. Verbindung nach Anspruch 1 für die Verwendung bei der Behandlung eines Säugetiers, welches unter einem Zustand leidet, welcher ausgewählt ist unter Hyperglykämie, Typ-2-Diabetes, gestörte Glukosetoleranz, Typ-1-Diabetes, Adipositas, Hypertension, Syndrom X, Dyslipidämie, kognitive Störungen, Atherosklerose, Myokardinfarkt, koronare Herzerkrankung und andere kardiovaskuläre Störungen, Schlaganfall, entzündliche Darmerkrankung, Dyspepsie und Magengeschwüre.

5. Verwendung einer Verbindung nach Anspruch 1 bei der Herstellung eines Arzneimittels für die Behandlung eines Zustands, welcher ausgewählt ist unter Hyperglykämie, Typ-2-Diabetes, gestörte Glukosetoleranz, Typ-1-Diabetes, Adipositas, Hypertension, Syndrom X, Dyslipidämie, kognitive Störungen, Atherosklerose, Myokardinfarkt, koronare Herzerkrankung und andere kardiovaskuläre Störungen, Schlaganfall, entzündliche Darmerkrankung, Dyspepsie und Magengeschwüre.

## Revendications

1. Composé de formule (XXV), (XXVII) ou (XXVIII) : 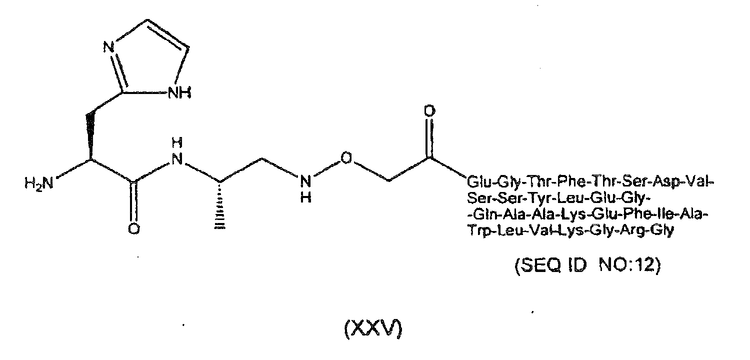 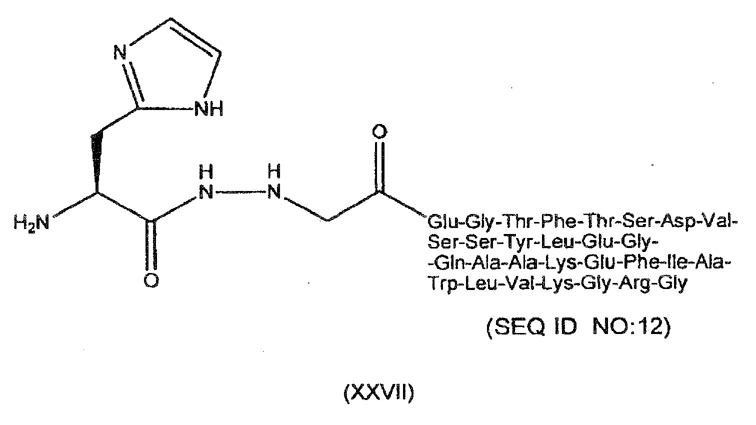 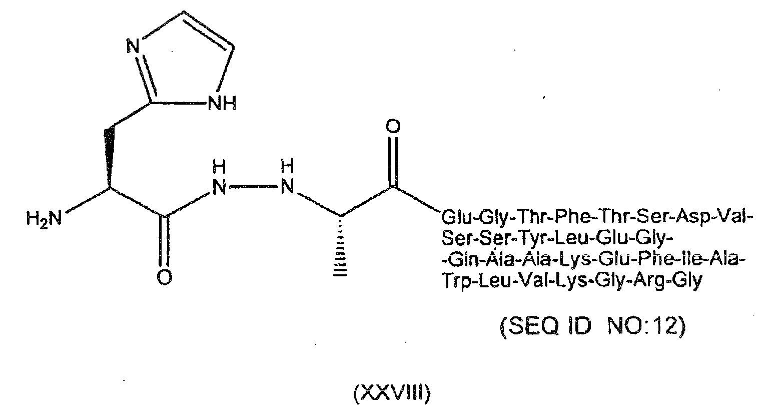 ou forme de sel pharmaceutiquement acceptable de celui-ci.

2. Composé selon la revendication 1 destiné à être utilisé comme médicament.

3. Composition pharmaceutique comprenant un composé selon la revendication 1 et un vecteur ou excipient pharmaceutiquement acceptable.

4. Composé selon la revendication 1 destiné à être utilisé dans le traitement d'un mammifère souffrant d'une affection choisie parmi l'hyperglycémie, le diabète de type 2, l'intolérance au glucose, le diabète de type 1, l'obésité, l'hypertension, le syndrome X, la dyslipidémie, les troubles cognitifs, l'athérosclérose, l'infarctus du myocarde, la maladie cardiaque coronarienne et d'autres troubles cardiovasculaires, l'attaque, le syndrome du côlon inflammatoire, la dyspepsie et les ulcères gastriques.

5. Utilisation d'un composé selon la revendication 1 dans la préparation d'un médicament pour le traitement d'une affection choisie parmi l'hyperglycémie, le diabète de type 2, l'intolérance au glucose, le diabète de type 1, l'obésité, l'hypertension, le syndrome X, la dyslipidémie, les troubles cognitifs, l'athérosclérose, l'infarctus du myocarde, la maladie cardiaque coronarienne et d'autres troubles cardiovasculaires, l'attaque, le syndrome du côlon inflammatoire, la dyspepsie et les ulcères gastriques.


### Sequence Listing

### SEQUENCE LISTING

<110> ACTIVOTEC SPP LIMITED
<120> Peptides and Uses Thereof
<130> P023799WO
<150> 0522295.5
   <151> 2005-11-01
<150> 0616061.8
   <151> 2006-08-11
<160> 13
<170> PatentIn version 3.3
<210> 1
   <211> 31
   <212> PRT
   <213> Homo sapiens
<400> 1
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 2
   <211> 29
   <212> PRT
   <213> Artificial
<220>
   <223> GLP-1 analogues
<220>
   <221> MISC_FEATURE
   <222> (1) .. (1)
   <223> Xaa at position 1 is chosen from His, D-histidine, desamino-histidine, 2-amino-histidine, beta-hydroxy-histidine, homohistidine, alpha-flouromethyl-histidine, and alpha-methyl-histidine.
<220>
   <221> MISC_FEATURE
   <222> (2).. (2)
   <223> Xaa at position 2 is chosen from Met, Asp, Lys, Thr, Leu, Asn, Gln, Phe, Val, and Tyr.
<220>
   <221> MISC_FEATURE
   <222> (15) .. (15)
   <223> Xaa at position 15 is chosen from Glu, Gln, Ala, Thr, Ser, and Gly.
<220>
   <221> misc_feature
   <222> (20)..(20)
   <223> Xaa can be any naturally occurring amino acid
<220>
   <221> MISC_FEATURE
   <222> (21) .. (21)
   <223> Xaa at position 21 is chosen from Glu, Gln, Ala, Thr, Ser, and Gly.
<220>
   <221> MISC_FEATURE
   <222> (30) .. (30)
   <223> Arg at position 30 is modified so as to replace the terminal carboxyl group with an amide, if Gly at position 31 is absent.
<400> 2
   Xaa Xaa Glu Gly Thr Phe Thr Ser Asp Val Ser Ser Tyr Leu Xaa Gln
   1               5                   10                  15
   Ala Ala Lys Xaa Phe Ile Ala Trp Leu Val Lys Gly Arg
               20                  25
<210> 3
   <211> 31
   <212> PRT
   <213> Artificial
<220>
   <223> GLP-1 analogues
<220>
   <221> MISC_FEATURE
   <222> (1) .. (1)
   <223> Xaa may be any amino acid.
<220>
   <221> MISC_FEATURE
   <222> (2) .. (2)
   <223> Xaa may be any amino acid.
<220>
   <221> MISC_FEATURE
   <222> (3) .. (3)
   <223> Xaa may be any amino acid.
<220>
   <221> MISC_FEATURE
   <222> (9) .. (9)
   <223> Xaa may be any amino acid.
<220>
   <221> MISC_FEATURE
   <222> (10) .. (10)
   <223> Xaa may be any amino acid.
<220>
   <221> MISC_FEATURE
   <222> (12) .. (12)
   <223> Xaa may be any amino acid.
<220>
   <221> MISC_FEATURE
   <222> (13) .. (13)
   <223> Xaa may be any amino acid.
<220>
   <221> MISC_FEATURE
   <222> (14) .. (14)
   <223> Xaa may be any amino acid.
<220>
   <221> MISC_FEATURE
   <222> (19) .. (19)
   <223> Xaa may be any amino acid.
<220>
   <221> MISC_FEATURE
   <222> (21) .. (21)
   <223> Xaa may be any amino acid.
<220>
   <221> MISC_FEATURE
   <222> (22) .. (22)
   <223> Xaa may be any amino acid.
<220>
   <221> MISC_FEATURE
   <222> (23)..(23)
   <223> Xaa may be any amino acid.
<220>
   <221> MISC_FEATURE
   <222> (25) .. (25)
   <223> Xaa may be any amino acid.
<220>
   <221> MISC_FEATURE
   <222> (26) .. (26)
   <223> Xaa may be any amino acid.
<220>
   <221> MISC_FEATURE
   <222> (30) .. (30)
   <223> Xaa may be any amino acid.
<400> 3
   Xaa Xaa Xaa Gly Thr Phe Thr Ser Xaa Xaa Ser Xaa Xaa Xaa Glu Gly
   1               5                   10                  15
   Gln Ala Xaa Lys Xaa Xaa Xaa Ala Xaa Xaa Val Lys Gly Xaa Gly
               20                  25                  30
<210> 4
   <211> 30
   <212> PRT
   <213> Artificial
<220>
   <223> GLP-1 analogues
<220>
   <221> MISC_FEATURE
   <222> (1) .. (1)
   <223> Xaa at position 1 is chosen from His, D-histidine, desamino-histidine, 2-amino-histidine, beta-hydroxy-histidine, homohistidine, alpha-flouromethyl-histidine, and alpha-methyl-histidine.
<220>
   <221> MISC_FEATURE
   <222> (2) .. (2)
   <223> Xaa at position 2 is chosen from Ala, Gly, Val, Thr, Ile and alpha-methyl-Ala.
<220>
   <221> MISC_FEATURE
   <222> (15) .. (15)
   <223> Xaa at position 15 is chosen from Glu, Gln, Ala, thr, Ser and Gly.
<220>
   <221> MISC_FEATURE
   <222> (21) .. (21)
   <223> Xaa at position 21 is chosen from Glu, Gln, Ala, thr, Ser and Gly.
<220>
   <221> MISC_FEATURE
   <222> (30) .. (30)
   <223> Arg at position 30 is modified so as to replace the terminal carboxyl group with an amide, if Gly at position 31 is absent. <400> 4
<210> 5
   <211> 37
   <212> PRT
   <213> Homo sapiens
<400> 5
   His Asp Glu Phe Glu Arg His Ala Glu Gly Thr Phe Thr Ser Asp Val
   1               5                   10                  15
   Ser Ser Tyr Leu Glu Gly Gln Ala Ala Lys Glu Phe Ile Ala Trp Leu
               20                  25                  30
   Val Lys Gly Arg Gly
           35
<210> 6
   <211> 29
   <212> PRT
   <213> Homo sapiens
<400> 6
   Glu Gly Thr Phe Thr Ser Asp Val Ser Ser Tyr Leu Glu Gly Gln Ala
   1               5                   10                  15
   Ala Lys Glu Phe Ile Ala Trp Leu Val Lys Gly Arg Gly
               20                  25
<210> 7
   <211> 5
   <212> PRT
   <213> Artificial
<220>
   <223> Preferred amino acid sequence.
<400> 7
   Gly Thr Phe Thr Ser
   1               5
<210> 8
   <211> 4
   <212> PRT
   <213> Artificial
<220>
   <223> Preferred amino acid sequence.
<400> 8
   Glu Gly Gln Ala
   1
<210> 9
   <211> 11
   <212> PRT
   <213> Artificial
<220>
   <223> Preferred amino acid sequence.
<400> 9
   Gly Thr Phe Thr Ser Asp Val Ser Ser Tyr Leu
   1               5                   10
<210> 10
   <211> 5
   <212> PRT
   <213> Artificial
<220>
   <223> Preferred amino acid sequence.
<400> 10
   Gly Gln Ala Ala Lys
   1 5
<210> 11
   <211> 10
   <212> PRT
   <213> Artificial
<220>
   <223> Preferred amino acid sequence.
<400> 11
   Phe Ile Ala Trp Leu Val Lys Gly Arg Gly
   1 5 10
<210> 12
   <211> 29
   <212> PRT
   <213> Artificial
<220>
   <223> Preferred amino acid sequence.
<400> 12
   Glu Gly Thr Phe Thr Ser Asp Val Ser Ser Tyr Leu Glu Gly Gln Ala
   1 5 10 15
   Ala Lys Glu Phe Ile Ala Trp Leu Val Lys Gly Arg Gly
   20 25
<210> 13
   <211> 28
   <212> PRT
   <213> Artificial
<220>
   <223> Preferred amino acid sequence.
<400> 13
   Glu Gly Thr Phe Thr Ser Asp Val Ser Ser Tyr Leu Glu Gly Gln Ala
   1               5                   10                  15
   Ala Lys Glu Phe Ile Ala Trp Leu Val Lys Gly Arg
               20                  25